# FASCICULE DE BREVET EUROPEEN

(11) **EP 1 353 929 B1**
(45) Date de publication et mention de la délivrance du brevet: **16.03.2005**
(21) Numéro de dépôt: 01995738.0
(22) Date de dépôt: 19.12.2001
(51) Int. Cl.: C07D 498/22, C07D 498/18, C07K 5/06, A61K 31/42, A61P 31/04

(54) **DERIVES DE STREPTOGRAMINES, LEUR PREPARATION ET LES COMPOSITIONS QUI LES CONTIENNENT**
STREPTOGRAMINDERIVATE, DEREN HERSTELLUNG UND ZUSAMMENSETZUNGEN, DIE DIESE VERBINDUNGEN ENTHALTEN
STREPTOGRAMIN DERIVATIVES PREPARATION THEREOF AND COMPOSITIONS CONTAINING SAME

(30) Priorité: 21.12.2000 FR 0016804
(43) Date de publication de la demande: 22.10.2003
(73) Titulaire: Aventis Pharma S.A., 92160 Antony (FR)
(72) Inventeur: DESMAZEAU, Pascal, F-91250 Tigery (FR); RONAN, Baptiste, F-92140 Clamart (FR); BACQUE, Eric, F-91190 Gif sue Yvette (FR); BARRIERE, Jean-Claude, F-91440 Bures sur Yvette (FR)
(86) Numéro de dépôt international: PCT/FR2001/004060
(87) Numéro de publication internationale: WO 2002/050084

(56) Documents cités:
- FR-A- 2 766 489
- GB-A- 2 301 821
- US-A- 5 242 938

## Description

La présente invention concerne des dérivés du groupe A des streptogramines de formule générale : 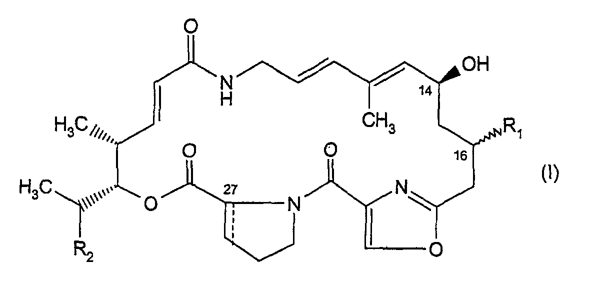 qui présentent une activité antibactérienne particulièrement intéressante.

Parmi les streptogramines connues, la pristinamycine (RP 7293), antibactérien d'origine naturelle produit par *Streptomyces pristinaespiralis* a été isolée pour la première fois en 1955. La pristinamycine commercialisée sous le nom de Pyostacine® est constituée principalement de pristinamycine IIA associée à la pristinamycine IA.

Un autre antibactérien de la classe des streptogramines : la virginiamycine, a été isolé à partir de *Streptomyces virginiae,* ATCC 13161 [Antibiotics and Chemotherapy, 5, 632 (1955)]. La virginiamycine (Staphylomycine®) est constituée principalement de facteur M₁ (VM1) associé au facteur S (VS).

Il a été maintenant trouvé, que les dérivés de streptogramine du groupe A de formule générale (I) dans laquelle :
- R₁ représente des radicaux cyano ou éthynyle,
- R₂ représente un atome d'hydrogène ou un radical méthyle ou éthyle, et
- la liaison --- représente une liaison simple (stéréochimie 27R) ou une liaison double,
présentent une activité antibactérienne particulièrement intéressante, seuls ou associés à un dérivé de streptogramine du groupe B.

Les dérivés de streptogramine de formule générale (I) peuvent être préparés à partir d'un dérivé sulfonyloxy-16 de formule générale : 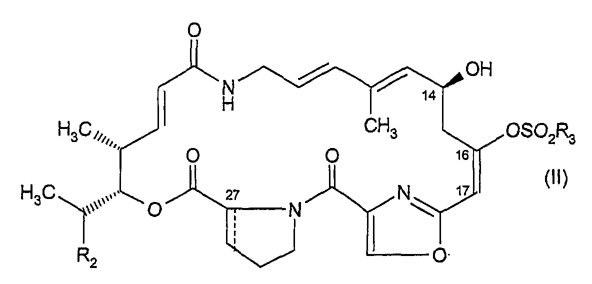 dans laquelle R₂ est défini comme précédemment, R₃ est un radical alcoyle perfluoré contenant 1 à 10 atomes de carbone et la liaison --- représente une liaison simple (stéréochimie 27R) ou une liaison double, soit par action d'un cyanure alcalin, suivie de la réduction du produit obtenu, soit par carbonylation, suivie de la réduction de la lactone formée en lactol et suivie de l'action du diazométhyl phosphonate de diméthyle ou du 1-diazo-2-oxopropyl phosphonate de diméthyle, puis éventuellement séparation des isomères 16R et 16S obtenus.

De préférence le reste fluoré d'un radical sulfonylé représenté par R₃ est choisi parmi trifluorométhyle ou nonafluorobutyle.

Le cyanure alcalin peut être avantageusement choisi parmi le cyanure de potassium, de sodium ou de césium.
La réaction de cyanation s'effectue en présence d'un dérivé du palladium (par exemple le palladium tetrakistriphénylphosphine) et d'iodure de cuivre. On opère dans un solvant organique tel qu'un nitrile (par exemple l'acétonitrile), un éther (par exemple le tétrahydrofuranne), un amide (par exemple diméthylformamide ou N-méthylpyrrolidinone) à une température comprise entre 20°C et la température de reflux du mélange réactionnel. De préférence on opère sous atmosphère inerte (argon, azote par exemple).

La réduction subséquente peut être mise en oeuvre par voie électrochimique, sous atmosphère inerte, à une température voisine de 20°C, en opérant dans une solution tampon notamment une solution dégazée sous argon, de tétrafluoroborate de tétraéthylammonium, d'acétate de tétraéthylammonium et d'acide acétique, sous une différence de potentiel d'environ -1,5 V (I=250mA). L'exemple 2 ci-après donne un aperçu plus détaillé des conditions opératoires pouvant être utilisées.

La réaction conduit au mélange des isomères 16R et 16S qui peuvent être séparés selon les méthodes habituelles qui n'altèrent pas le reste de la molécule, par exemple par chromatographie [Chromatographie Liquide Haute Performance (CLHP) sur phase normale ou inverse, sur phase chirale ou non ou par chromatographie flash] ou par cristallisation.

La réaction de carbonylation peut être mise en oeuvre sous atmosphère de monoxyde de carbone (de préférence sous 1 atmosphère) en présence d'un dérivé du palladium, par exemple le palladium tetrakistriphénylphosphine, de chlorure de lithium anhydre et d'une base comme un carbonate alcalin ou alcalino-terreux (par exemple le carbonate de potassium de sodium ou de césium), dans un solvant organique inerte tel qu'un éther (par exemple le tétrahydrofuranne), un nitrile (par exemple l'acétonitrile), un amide (par exemple le diméthylformamide ou la N-méthylpyrrolidinone) à une température comprise entre 0°C et la température de reflux du mélange réactionnel. De préférence on opère à environ 20°C.

La réaction de carbonylation conduit à la formation de la lactone de formule générale : 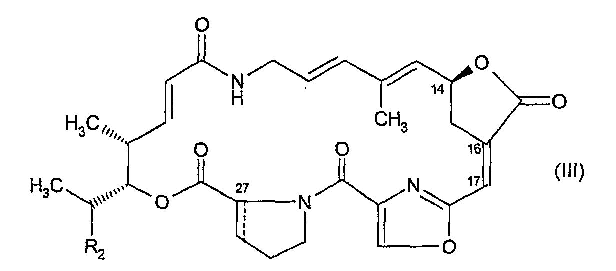 dans laquelle R₂ est défini comme précédemment et la liaison --- représente une liaison simple (stéréochimie 27R) ou une liaison double. Cette lactone est réduite en lactol saturé en 16,17 puis soumise à la réaction du diazométhyl phosphonate de diméthyle ou du 1-diazo-2-oxopropyl phosphonate de diméthyle pour former le dérivé éthynyle.

La réduction de la lactone obtenue en lactol peut être effectuée en présence d'un hydrure comme par exemple le tri-sec-butyl borohydrure de lithium ou de potassium (L ou K Selectride), dans un solvant organique inerte comme un éther (par exemple le tétrahydrofuranne) à une température comprise entre -60 et 20°C, de préférence aux environs de -20°C et sous atmosphère inerte (azote ou argon).

La réaction du diazométhyl phosphonate de diméthyle ou du 1-diazo-2-oxopropyl phosphonate de diméthyle s'effectue par application des méthodes décrites par H.J. Bestmann et coll., Synlett, 521 (1996) ou par K.C. Nicolaou, Tetrahedron, 50(39), 11391 (1994) incorporées ici à titre de référence ; notamment en opérant dans un solvant inerte comme un éther (par exemple le tétrahydrofuranne), un alcool (méthanol par exemple), à une température comprise entre -78 et 40°C, de préférence à environ 20°C. On opère avantageusement sous atmosphère inerte (argon, azote par exemple).

La réaction conduit également au mélange des isomères 16R et 16S qui peuvent être séparés selon les méthodes habituelles qui n'altèrent pas le reste de la molécule, par exemple par chromatographie [Chromatographie Liquide Haute Performance (CLHP) sur phase normale ou inverse, sur phase chirale ou non ou par chromatographie flash] ou par cristallisation.

Le dérivé de la streptogramine de formule générale (II) peut être obtenu par action de du fluorure ou de l'anhydride correspondant sur un dérivé de la streptogramine de formule générale : 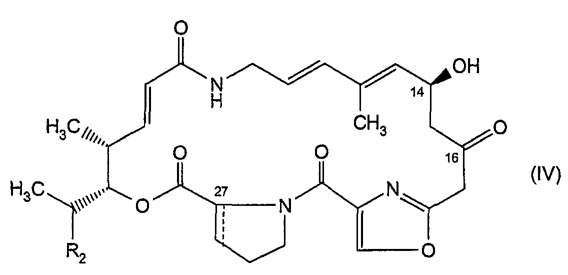 dans laquelle R₂ est défini comme précédemment et la liaison --- représente une liaison simple (stéréochimie 27R) ou une liaison double, en opérant en présence d'une base comme une amine tertiaire (diisopropyléthylamine, triéthylamine par exemple), dans un solvant organique inerte comme par exemple un solvant chloré (dichlorométhane par exemple), un éther (par exemple le tétrahydrofuranne), un amide (par exemple diméthylformamide) à une température comprise entre -78 et 20°C, de préférence aux environs de -70°C et sous atmosphère inerte (argon, azote par exemple).

On fait avantageusement agir l'anhydride sulfonique (anhydride triflique par exemple) ou le fluorure de sulfonyle (par exemple fluorure de perfluoro-1-butanesulfonyle) correspondants au radical R₃ choisi.

Les dérivés de pristinamycine de formule générale (IV) correspondent respectivement à la pristinamycine IIA (PIIA), à la pristinamycine IIB (PIIB), à la pristinamycine IIC (PIIC), à la pristinamycine IID (PIID), à la pristinamycine IIF (PIIF) et à la pristinamycine IIG (PIIG) qui sont des composantes connues de la pristinamycine naturelle. Les composantes PIIF et PIIG ont été décrites dans le brevet européen EP 614910. La pristinamycine IIC (PIIC), et la pristinamycine IID (PIID) peuvent être obtenues comme décrit par J.C. Barrière et coll., Expert. Opin. Invest. Drugs, 3(2), 115-31 (1994).

La préparation et la séparation des composantes des streptogramines naturelles du groupe A [streptogramines de formule générale (IV)] est effectuée par fermentation et isolement des constituants à partir du môut de fermentation selon ou par analogie avec la méthode décrite par J. Preud'homme et coll., Bull. Soc. Chim. Fr., vol. 2, 585 (1968) ou dans le brevet européen EP 614910. Alternativement la préparation des composantes naturelles du groupe A peut être effectuée par fermentation spécifique, comme décrit dans la demande de brevet FR 2 689 518.

Les dérivés de streptogramine du groupe A, de formule générale (II) et (III) sont des produits nouveaux utiles pour la préparation des dérivés de streptogramine selon l'invention.

Les dérivés de streptogramine de formule générale (I) peuvent être purifiés le cas échéant par des méthodes physiques telles que la cristallisation, la chromatographie ou la CPC.

Les dérivés de streptogramine selon la présente invention présentent des propriétés antibactériennes et des propriétés synergisantes de l'activité antibactérienne des dérivés de streptogramine du groupe B. Ils sont particulièrement intéressants du fait de leur activité puissante seuls ou associés.

Lorsqu'ils sont associés avec une composante ou un dérivé du groupe B des streptogramines, ces derniers peuvent être choisis selon que l'on désire obtenir une forme administrable par voie orale ou parentérale, parmi les composantes naturelles : pristinamycine IA, pristinamycine IB, pristinamycine IC, pristinamycine ID, pristinamycine IE, pristinamycine IF, pristinamycine IG, virginiamycine S1, S3 ou S4, vernamycine B ou C, étamycine ou parmi des dérivés d'hémisynthèse tels que décrits dans les brevets ou demandes de brevet US 4618599, US 4798827, US 5326782, EP 772630 ou EP 770132, notamment des dérivés de streptogramines de formule générale : 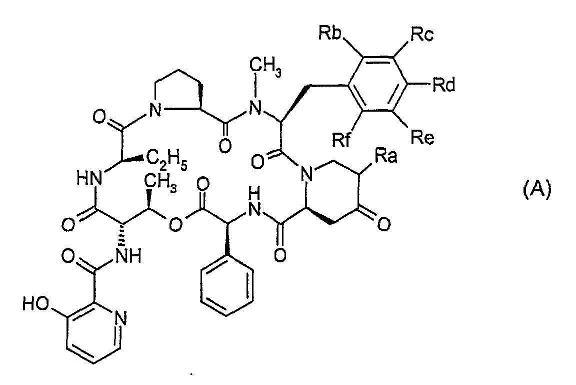 dans laquelle,
1. Rb, Rc, Re et Rf sont des atomes d'hydrogène, Rd est un atome d'hydrogène ou un radical diméthylamino, et Ra est un radical de structure -CH₂R'a pour lequel R'a est pyrrolidinyl-3thio, pipéridyl-3(ou -4)thio pouvant être substitués par alcoyle, alcoylthio substitué par 1 ou 2 hydroxysulfonyle, alcoylamino, dialcoylamino (lui même éventuellement substitué par mercapto ou dialcoylamino), ou substitué par 1 ou 2 cycles pipérazine éventuellement substitué, morpholino, thiomorpholino, pipéridino, pyrrolidinyle-1, pipéridyle-2,-3 ou -4, ou pyrrolidinyle-2 ou -3 (pouvant être substitués par alcoyle), ou bien Ra est un radical de structure =CHR'a pour lequel R'a est pyrrolidinyl-3amino, pipéridyl-3(ou -4)amino, pyrrolidinyl-3oxy, pipéridyl-3(ou -4)oxy, pyrrolidinyl-3thio, pipéridyl-3(ou -4)thio pouvant être substitués par alcoyle, ou R'a est alcoylamino, alcoyloxy ou alcoylthio substitués par 1 ou 2 hydroxysulfonyle, alcoylamino, dialcoylamino (lui même éventuellement substitué par dialcoylamino), ou par trialcoylammonio, imidazolyl-4 ou -5, ou par 1 ou 2 cycles pipérazine éventuellement substitué, morpholino, thiomorpholino, pipéridino, pyrrolidinyle-1, pipéridyle-2,-3 ou -4, ou pyrrolidinyle-2 ou -3 (pouvant être substitués par alcoyle), ou
   Ra est un radical quinuclidinyl-3(ou -4)thiométhyle ou bien
2. Ra est un atome d'hydrogène et
   a) soit Rb, Re et Rf sont des atomes d'hydrogène, Rd est un radical -NHCH₃ ou -N(CH₃)₂ et Rc est un atome de chlore ou de brome, ou représente un radical alcényle contenant 3 à 5 atomes de carbone [si Rd est -N(CH₃)₂],
   b) soit Rb, Rd, Re et Rf représentent un atome d'hydrogène et Rc est un halogène, ou un radical aminomonoalkyle, aminodialkyle, alcoyloxy, trifluorométhyloxy, thioalcoyle,alcoyle en C₁ à C₃ ou trihalogénométhyle
   c) soit Rb, Rc, Re et Rf représentent un atome d'hydrogène et Rd est un halogène, ou un radical éthylamino, diéthylamino ou méthyléthylamino, alcoyloxy ou trifluorométhyloxy, thioalkyle, alcoyle en C₁ à C₆, aryle ou trihalogénométhyle
   d) soit Rb, Re et Rf représentent un atome d'hydrogène et Rc est halogène ou un radical aminomonoalkyle ou aminodialkyle, alcoyloxy ou trifluorométhyloxy, thioalkyle, alcoyle en C₁ à C₃, et Rd est halogène ou un radical amino, aminomonoalkyle ou aminodialkyle, alcoyloxy ou trifluorométhyloxy, thioalkyle, alcoyle en C₁ à C₆ ou trihalogénométhyle,
   e) soit Rc, Re et Rf représentent un atome d'hydrogène et Rb et Rd représentent un radical méthyle ;
ou encore parmi les dérivés d'hémisynthèse du groupe B des streptogramines de formule générale : 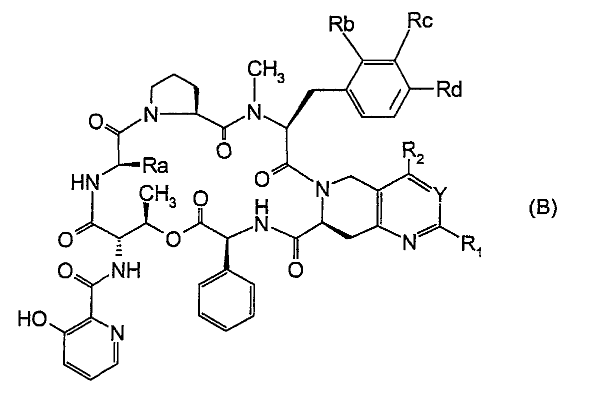 dans laquelle
Y est un atome d'azote ou un radical =CR₃-,
R₁ est un atome d'hydrogène, un radical alcoyle (1 à 8 carbones), alcényle (2 à 8 carbones), cycloalcoyle (3 à 8 carbones), hétérocyclyle saturé ou insaturé (3 à 8 chaînons), phényle, phényle substitué [par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino] ou un radical NR'R", R' et R" identiques ou différents pouvant être des atomes d'hydrogène ou des radicaux alcoyle (1 à 3 carbones), ou pouvant former ensemble avec l'atome d'azote auquel ils sont attachés, un hétérocycle de 3 à 8 chaînons contenant éventuellement un autre hétéroatome choisi parmi l'oxygène le soufre ou l'azote éventuellement substitué [par un radical alcoyle, alcényle (2 à 8 carbones), cycloalcoyle (3 à 6 carbones), hétérocyclyle saturé
ou insaturé (4 à 6 chaînons), benzyle, phényle ou phényle substitué tel que défini ci-dessus pour la définition de R₁]
ou bien lorsque Y est un radical =CR₃-, R₁ peut être aussi halogénométhyle, hydroxyméthyle, alcoyloxyméthyle, alcoylthiométhyle dont la partie alcoyle est éventuellement substituée par NR'R", alcoylsulfmylméthyle, alcoylsulfonylméthyle, acyloxyméthyle, benzoyloxyméthyle, cyclopropylaminométhyle ou -(CH₂)ₙNR'R" (n étant un entier de 1 à 4 et R' et R" étant définis comme ci-dessus), ou bien si R₃ est un atome d'hydrogène, R₁ peut être aussi formyle, carboxy, alcoyloxycarbonyle,
ou -CONR'R" pour lequel R' et R" sont définis comme ci-dessus,
ou bien lorsque Y est un atome d'azote, R₁ peut être aussi un radical -XR° pour lequel X est un atome d'oxygène ou de soufre, un radical sulfinyle ou sulfonyle, ou un radical NH et R° est un radical alcoyle (1 à 8 carbones), cycloalcoyle (3 à 6 carbones), hétérocyclyle saturé ou insaturé (3 à 8 chaînons), hétérocyclylméthyle (3 à 8 chaînons) dont la partie hétérocyclyle est attachée au radical méthyle par un atome de carbone, phényle, phényle substitué [par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino] ou un radical -(CH₂)ₙNR'R" pour lequel R' et R" sont définis comme ci-dessus et n est un entier de 2 à 4, ou bien, si X représente NH, R° peut aussi représenter l'atome d'hydrogène,
R₂ est un atome d'hydrogène ou un radical alcoyle (1 à 3 carbones),
R₃ est un atome d'hydrogène ou un radical alcoyle, carboxy, alcoyloxycarbonyle ou carbamoyle de structure -CO-NR'R" dans laquelle R' et R" sont définis comme précédemment,
Ra est un radical méthyle ou éthyle, et
Rb, Rc et Rd ont les définitions ci-après :
1) Rb et Rc sont des atomes d'hydrogène et Rd est un atome d'hydrogène ou un radical méthylamino ou diméthylamino,
2) Rb est un atome d'hydrogène, Rc est un atome d'hydrogène, de chlore ou de brome, ou représente un radical alcényle (3 à 5C), et Rd est un radical -NMe-R"' pour lequel R"' représente un radical alcoyle, hydroxyalcoyle (2 à 4C), ou alcényle (2 à 8C) éventuellement substitué par phényle, cycloalcoyl(3 à 6C)méthyle, benzyle, benzyle substitué [par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino], hétérocyclylméthyle ou hétérocyclyléthyle dont la partie hétérocyclyle est saturée ou insaturée et contient 5 à 6 chaînons et 1 ou 2 hétéroatomes choisis parmi le soufre, l'oxygène ou l'azote éventuellement substitué [par un radical alcoyle, alcényle (2 à 8 carbones), cycloalcoyle (3 à 6 carbones), hétérocyclyle saturé ou insaturé (4 à 6 chaînons), phényle, phényle substitué tel que défini ci-avant pour la définition de R₁ ou benzyle], ou bien R"' représente un radical cyanométhyle, ou -CH₂CORe pour lequel soit Re est -OR'e, R'e étant hydrogène, alcoyle (1 à 6 carbones), alcényle (2 à 6 carbones), benzyle ou hétérocyclylméthyle dont la partie hétérocyclyle contient 5 à 6 chaînons et 1 ou 2 hétéroatomes choisis parmi le soufre, l'oxygène ou l'azote soit Re est un radical alcoylamino, alcoyl méthyl amino, hétérocyclylamino ou hétérocyclyl méthyl amino dont la partie hétérocyclyle est saturée et contient 5 à 6 chaînons et 1 ou 2 hétéroatomes choisis parmi le soufre, l'oxygène ou l'azote éventuellement substitué par un radical alcoyle, benzyle ou alcoyloxycarbonyle,
3) Rb est un atome d'hydrogène, Rd est un radical -NHCH₃ ou -N(CH₃)₂ et Rc est un atome de chlore ou de brome, ou représente un radical alcényle (3 à 5C), [si Rd est -N(CH₃)₂],
4) Rb et Rd sont des atomes d'hydrogène et Rc est un atome d'halogène, ou un radical alcoylamino ou dialcoylamino, alcoyloxy, trifluorométhoxy, thioalcoyle, alcoyle (1 à 6C) ou trihalogénométhyle,
5) Rb et Rc sont des atomes d'hydrogène et Rd est un atome d'halogène, ou un radical éthylamino, diéthylamino ou méthyl éthyl amino, alcoyloxy ou trifluorométhoxy, alcoylthio, alcoylsulfinyle, alcoylsulfonyle, alcoyle (1 à 6C), phényle ou trihalogénométhyle,
6) Rb est un atome d'hydrogène et Rc est un atome d'halogène ou un radical alcoylamino ou dialcoylamino, alcoyloxy ou trifluorométhoxy, thioalcoyle, alcoyle (1 à 3C), et Rd est un atome d'halogène ou un radical amino, alcoylamino ou dialcoylamino, alcoyloxy ou trifluorométhoxy, thioalcoyle, alcoyle (1 à 6C) ou trihalogénométhyle,
7) Rc est un atome d'hydrogène et Rb et Rd représentent un radical méthyle, ainsi que leurs sels,
ou encore parmi les dérivés d'hémisynthèse du groupe B des streptogramines de formule générale : 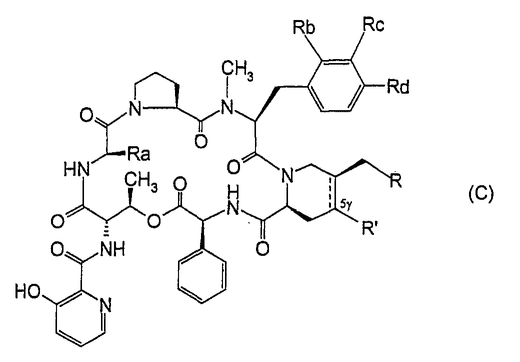 dans laquelle R représente un radical -NR₁R₂ ou -SR₃ [pour lequel R₁ et R₂ identiques ou différents représentent H, alcoyle (1 à 8C) éventuellement substitué par OH, alcényle (3 à 8C), cycloalcoyle (3 à 8C), alcoyloxy (1 à 8C), dialcoylamino, phénylalcoyle éventuellement substitué [par un ou plusieurs halogène ou alcoyle, hydroxyalcoyle, alcoyloxy ou dialcoylamino], hétérocyclylalcoyle saturé ou insaturé (3 à 8 chaînons) contenant 1 ou plusieurs hétéroatomes choisis parmi N, S ou O, ou bien R₁ et R₂ forment ensemble avec l'atome d'azote, un hétérocycle mono ou polycyclique, saturé, partiellement saturé ou insaturé (3 à 12 chaînons), contenant éventuellement un autre hétéroatome choisi parmi N, S ou O, et éventuellement substitué [par un ou plusieurs OH, alcoyle, phényle éventuellement substitué par un atome d'halogène, phénylalcoyle, phénylalcényle (alcényle contenant 2 à 4C), hydroxyalcoyle, acyle, alcoyloxycarbonyle, ou hétérocyclyle ou hétérocyclylcarbonyle dont la partie hétérocyclyle est saturée ou insaturée (4 à 6 chaînons) et contient 1 ou plusieurs hétéroatomes choisis parmi N, S ou O,], R₃ est alcoyle (1 à 8C) ou cycloalcoyle (3 à 8C) substitués par -NR₁R₂ pour lequel R₁ et R₂ identiques ou différents sont H ou alcoyle ou forment ensemble avec l'atome d'azote auquel ils sont attachés, un hétérocycle tel que défini ci-dessus, ou bien R₃ est hétérocyclyle ou hétérocyclylméthyle saturé ou insaturé (3 à 7 chaînons) et éventuellement un autre hétéroatome choisi parmi l'oxygène le soufre ou l'azote et éventuellement substitué par un radical alcoyle ; 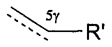 représente le reste d'un cycle insaturé non substitué en 5γ : 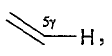 ou le reste d'un cycle saturé substitué en 5γ par un radical fluoro : 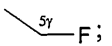 Ra est un radical Me ou Et, et Rb, Rc et Rd sont définis ci-après :
1) Rb et Rc sont H et Rd est H ou un radical MeNH ou NMe₂,
2) Rb est H, Rc est H, Cl ou Br, ou alcényle (3 à 5C), et Rd est -NMe-R"', R'" étant alcoyle, hydroxyalcoyle (2 à 4C), ou alcényle (2 à 8C), phénylalcényle, cycloalcoyl(3 à 6C)méthyle, benzyle, benzyle substitué, hétérocyclylméthyle, hétérocyclyléthyle, ou bien R"' est -CH₂CN, -CH₂COOH ou -CORe ou -CH₂CORe pour lesquels soit Re est -OR'e, soit Re est alcoylamino, alcoyl méthyl amino, hétérocyclylamino ou hétérocyclyl méthyl amino,
3) Rb est H, Rd est un radical -NHCH₃ ou -N(CH₃)₂ et Rc est Cl ou Br, ou alcényle (3 à 5C), [si Rd est -N(CH₃)₂],
4) Rb et Rd sont H et Rc est halogène, ou alcoylamino ou dialcoylamino, alcoyloxy, trifluorométhoxy, thioalcoyle, alcoyle (1 à 6C) ou trihalogénométhyle,
5) Rb et Rc sont H et Rd est halogène, ou éthylamino, diéthylamino ou méthyl éthyl amino, alcoyloxy ou trifluorométhoxy, alcoylthio, alcoylsulfinyle, alcoylsulfonyle, alcoyle (1 à 6C), phényle ou trihalogénométhyle,
6) Rb est H et Rc est halogène ou alcoylamino ou dialcoylamino, alcoyloxy ou trifluorométhoxy, thioalcoyle, alcoyle (1 à 3C), et Rd est halogène ou un radical amino, alcoylamino ou dialcoylamino, alcoyloxy ou trifluorométhoxy, thioalcoyle, alcoyle (1 à 6C) ou trihalogénométhyle
7) Rc est H et Rb et Rd sont CH₃
ainsi que ses sels lorsqu'ils existent.

Il est entendu que les associations formées au moyen des dérivés selon l'invention et des streptogramines du groupe B entrent également dans le cadre de la présente invention.

Les dérivés du groupe B des streptogramines de structure (B) peuvent être préparés selon les méthodes décrites dans la demande internationale PCT/FR 99/00409. Les dérivés du groupe B des streptogramines de structure (C) peuvent être préparés selon les méthodes décrites dans la demande internationale PCT/FR 00/02146.

In vitro sur *Staphylococcus aureus IP8203,* les dérivés de streptogramine selon l'invention se sont montrés actifs à des concentrations comprises entre 0,015 et 32 µg/ml seuls ou associés à un dérivé du groupe B comme la pristinamycine IB; in vivo, ils synergisent l'activité antimicrobienne de la pristinamycine I_{B} ou de dérivés d'hémisynthèse de formule générale (C) sur les infections expérimentales de la souris *à Staphylococcus aureus IP8203* à des doses comprises entre 72 et 150 mg/kg par voie orale (DC₅₀).

Enfin, les produits selon l'invention sont particulièrement intéressants du fait de leur faible toxicité. Aucun des produits n'a manifesté de toxicité à des doses de 150 mg/kg sur *Staphylococcus aureus IP8203,* 2 fois par jour, par voie sous-cutanée ou par voie orale chez la souris.

Les dérivés de streptogramine de formule générale (I) pour lesquels :
- R₁ représente un radical cyano ou éthynyle,
- R₂ représente un radical méthyle, et
- la liaison --- représente une liaison simple (stéréochimie 27R) ou une liaison double,
sont particulièrement intéressants.

Et parmi ces produits tout spécialement les produits décrits ci-après dans les exemples.

Parmi les dérivés du groupe A des streptogramines selon l'invention, on citera également les produits de formule générale (I) suivants :
. (16*R*)-16-Désoxo-16-éthynyl pristinamycine II_{A}
. (16S)-16-Désoxo-16-éthynyl pristinamycine II_{A}
. (16*R*)-16-cyano-16-Désoxo pristinamycine II_{A}
. (16S)-16-cyano-i6-Désoxo pristinamycine II_{A}.

Les exemples suivants, donnés à titre non limitatif, illustrent la présente invention.

Dans les exemples qui suivent, la nomenclature 16-désoxo pristinamycine IIA (ou ITB) signifie le remplacement de la fonction cétone en position 16 par 2 atomes d'hydrogène. Au fur et à mesure de la chromatographie, toutes les fractions sont analysées par chromatographie en couche mince (CCM), sur plaques de silice Merck 60F254. Les fractions correspondants à un même tâche en CCM sont regroupées puis concentrées à sec, sous pression réduite (30°C; 2,7 kPa). Les résidus ainsi obtenus sont analysés par les techniques spectroscopiques habituelles (RMN; IR; MS) ce qui permet d'identifier le produit attendu.

### Exemple 1

### 16-Désoxo-16-éthynyl pristinamycine II_{B} : (mélange des isomères 16R et 16S dans les proportions 70 / 30)

A 0,8 g de (16*R*)-16-désoxo-14,16-butyrolactol pristinamycine II_{B} en solution dans 40 cm³ de méthanol, on ajoute à 20°C, sous atmosphère d'argon, 0,415 g de carbonate de potassium et 0,35 g de 1-diazo-2-oxopropyl phosphonate de diméthyle. Après une nuit d'agitation, le mélange réactionnel est dilué par 100 cm³ de dichlorométhanepuis lavé par 70 cm³ d'une solution aqueuse saturée de bicarbonate de sodium. La phase organique est décantée, concentrée sous pression réduite (2,7 kPa) jusqu'à 30 cm³ et le résidu dilué par ajout de 70 cm³ de dichlorométhane. La solution obtenue est lavée par 40 cm³ d'eau. La phase organique est décantée, séchée sur sulfate de magnésium, filtrée puis concentrée sous pression réduite à sec (2,7 kPa) pour donner 0,8 g d'une meringue jaune pâle qui est purifiée par chromatographie-flash sur silice [éluant : dichlorométhane / acétonitrile / méthanol (95 / 2,5 / 2,5 en volumes)]. Après concentration des fractions contenant l'attendu, on obtient un solide qui est agité dans 15 cm³ de diisopropyl oxyde puis filtré et séché sous pression réduite (2,7 kPa) pour donner 0,27 g de 16-désoxo-16-éthynyl pristinamycine II_{B} (mélange des isomères 16*R* et 16*S* dans les proportions 70 / 30), sous forme d'un solide blanc fondant vers 128°C avec décomposition.

Spectre de R.M.N. ¹H (300 MHz, CDCl₃, δ en ppm). Nous observons le mélange des isomères (16R) et (16S) dans les proportions 70/30.

0,96 (d, J = 6,5 Hz : 3H) ; 1,00 (mt : 3H) ; 1,09 (mt : 3H) ; de 1,45 à 1,60 et 1,70 à 2,30 (2 séries de mt : 7H) ; 1,82 et 1,87 (2 s : 3H en totalité) ; 2,14 et 2,24 (2 d, J = 2 Hz : 1H en totalité) ; 2,75 (mt : 1H) ; de 2,75 à 2,90 et de 2,95 à 3,15 (2 séries de mt : 3H en totalité) ; de 3,35 à 3,65 (mt : 1H en totalité) ; de 3,75 à 3,95 et 4,08 (2 séries de mt : 2H en totalité) ; 4,50 (mt : 1H) ; de 4,70 à 4,90 (mt : 3H) ; 5,35 et 5,52 (2 d larges, J = 9 Hz : 1H en totalité) ; de 5,60 à 5,85 (mt : 1H) ; 5,83 (dd, J = 16,5 et 1,5 Hz : 1H) ; 5,90 et 5,99 (2 mts : 1H en totalité); 6,18 et 6,20 (2 d, J = 16 Hz : 1H en totalité) ; 6,48 et 6,51 (2 dd, J = 16,5 et 5 Hz : 1H en totalité) ; 8,09 et 8,11 (2 s : 1H en totalité).

Les isomères 16*R* et 16*S* peuvent être séparés par chromatographie liquide haute performance sur phase stationnaire silice (colonne préparative : 80 x 350 mm ; phase stationnaire : Silice Hypersil 8 µm ; phase mobile : CH₂Cl₂ 46 % + heptane 50 % + MeOH 2 % + CH₃CN 2 % ; débit : 100 ml / mn ; détection : UV 265 nm). Une solution de 1,1 g de 16-désoxo-16-éthynyl pristinamycine II_{B} (mélange des isomères 16*R* et 16*S* dans les proportions 70 / 30) dans 100 cm³ de dichlorométhane est injectée sur la colonne préparative. L'opération complète est renouvelée une seconde fois sur une solution identique. Après concentration des fractions contenant l'isomère A, on obtient 0,99 g de l'isomère A de la 16-désoxo-16-éthynyl pristinamycine II_{B} sous forme d'une poudre blanche.

**Isomère A :** Spectre de R.M.N. ¹H (400 MHz, CDCl₃, δ en ppm) : 0,97 (d, J = 6,5 Hz : 3H) ; 1,01 (d, J = 6,5 Hz : 3H) ; 1,10 (d, J = 6,5 Hz : 3H) ; 1,56 (mt : 1H) ; de 1,75 à 2,05 (mt : 5H) ; 1,87 (s : 3H) ; de 2,05 à 2,15 (mt : 1H) ; 2,15 (d, J = 2 Hz : 1H) ; 2,77 (mt : 1H) ; 2,81 (dd, J = 16 et 8 Hz : 1H) ; 3,04 (mt : 1H) ; 3,10 (dd, J = 16 et 6,5 Hz : 1H) ; 3,54 (mt : 1H) ; 3,88 (mt : 1H) ; 4,09 (mt : 1H) ; 4,52 (mt : 1H) ; de 4,75 à 4,85 (mt : 3H) ; 5,36 (d large, J = 9 Hz : 1H) ; 5,76 (ddd, J = 16 - 8 et 4Hz : 1H) ; 5,83 (d large, J = 16,5 Hz : 1H) ; 6,02 (mt : 1H) ; 6,21 (d, J = 16 Hz : 1H) ; 6,52 (dd, J = 16,5 et 5 Hz : 1H) ; 8,10 (s : 1H).

Après concentration des fractions contenant l'isomère B, on obtient 0,5 g de l'isomère B de la 16-désoxo-16-éthynyl pristinamycine II_{B} sous forme d'une poudre blanche.

**Isomère B :** Spectre de R.M.N. ¹H (400 MHz, CDCl₃, δ en ppm) : 0,97 (d, J = 6,5 Hz : 3H) ; 1,01 (d, J = 6,5 Hz : 3H) ; 1,09 (d, J = 6,5 Hz : 3H) ; de 1,70 à 2,05 (mt : 5H) ; 1,83 (s : 3H) ; de 2,05 à 2,20 (mt : 2H) ; 2,25 (d, J = 2 Hz : 1H) ; 2,76 (mt : 1H) ; de 3,00 à 3,15 (mt : 3H) ; 3,40 (mt : 1H) ; de 3,80 à 4,00 (mt : 2H) ; 4,51 (mt : 1H) ; de 4,70 à 4,85 (mt : 2H) ; 4,87 (mt : 1H) ; 5,54 (d large, J = 9 Hz : 1H) ; 5,67 (mt : 1H) ; 5,79 (d large, J = 16,5 Hz : 1H) ; 5,91 (mt : 1H) ; 6,19 (d, J = 16 Hz : 1H) ; 6,49 (dd, J = 16,5 et 5 Hz : 1H) ; 8,12 (s : 1H).

Le 1-diazo-2-oxopropyl phosphonate de diméthyle peut être préparé selon Callant et *al*, Synth. Comm. (1984) p155.

La (16*R*)-16-désoxo-14,16-butyrolactol pristinamycine II_{B} peut être obtenue de la façon suivante.

A 4,18 g de 16,17-déhydro-16-désoxo-14,16-butyrolactone pristinamycine II_{B} en suspension dans 84 cm³ de tétrahydrofuranne, on ajoute lentement à -20°C, sous atmosphère d'argon, 15,5 cm³ de L-Selectride®. Après 1 heure d'agitation, on ajoute 1,7 cm³ d'acide acétique puis 450 cm³ d'eau. Le mélange réactionnel est extrait par 3 fois 120 cm³ d'acétate d'éthyle. Les phases organiques sont regroupées, lavées avec 150 cm³ d'une solution aqueuse saturée de chlorure de sodium, séchées sur sulfate de magnésium, filtrées puis concentrées sous pression réduite à sec (2,7 kPa) pour donner, après agitation dans 150 cm³ de diéthyl oxyde, une meringue blanche. Après purification par chromatographie-flash sur silice [éluant : dichlorométhane / acétonitrile / méthanol (96 / 2 / 2 en volumes)] et concentration des fractions contenant l'attendu, on obtient 1,8 g de (16*R*)-16-désoxo-14,16-butyrolactol pristinamycine II_{B}, sous forme d'un solide blanc.

Spectre de R.M.N. ¹H (400 MHz, CDCl₃, δ en ppm) : 0,99 (d, J = 6,5 Hz : 3H) ; 1,07 (d, J = 6,5 Hz : 3H) ; 1,10 (d, J = 6,5 Hz : 3H) ; de 1,65 à 2,25 (mt : 6H) ; 1,73 (s : 3H) ; 2,47 (mt : 1H) ; 2,66 (mt : 1H) ; 2,79 (mt : 1H) ; 2,84 (dd, J = 16 et 4 Hz : 1H) ; 3,01 (dd, J = 16 et 7 Hz : 1H) ; 3,56 (mt : 1H) ; 4,03 (mt: 1H); 4,10 (mt : 1H) ; 4,56 (mt : 1H) ; 4,70 (dd, J = 8 et 3 Hz : 1H) ; 4,74 (dd, J = 10 et 1,5 Hz : 1H) ; 5,08 (mt : 1H) ; de 5,35 à 5,45 (mt : 2H) ; 5,74 (ddd, J = 16 - 5 et 4 Hz : 1H) ; 5,83 (dd, J = 16,5 et 1,5 Hz : 1H) ; 6,10 (mt : 1H) ; 6,19 (d, J = 16 Hz : 1H) ; 6,52 (dd, J = 16,5 et 5 Hz : 1H) ; 8,17 (s : 1H).

La 16,17-déhydro-16-désoxo-14,16-butyrolactone pristinamycine II_{B} peut être préparée de la manière suivante :
A 21 g de 16,17-déhydro-16-trifluorométhanesulfonyloxy pristinamycine II_{B} en solution dans 250 cm³ de tétrahydrofuranne, on ajoute à 20°C, sous atmosphère d'argon, 1,47 g de palladium tétrakistriphénylphosphine, 3,37 g de chlorure de lithium et 8,8 g de carbonate de potassium. On fait buller du monoxyde de carbone au sein du mélange réactionnel pendant 27 heures avant ajout de 4,4 g de carbonate de potassium et continuation du bullage du monoxyde de carbone dans le mélange réactionnel durant 68 heures. Le mélange réactionnel est filtré sur Célite® , dilué avec 250 cm³ de dichlorométhane et agité avec 200 cm³ d'une solution aqueuse saturée de chlorure de sodium. Après filtration sur Célite® , la phase organique est lavée par 2 fois 200 cm³ d'une solution aqueuse saturée de chlorure de sodium, séchée sur sulfate de magnésium, filtrée puis concentrée sous pression réduite à sec (2,7 kPa) pour donner 13,5 g de 16,17-déhydro-16-désoxo-14,16-butyrolactone pristinamycine II_{B} sous la forme d'un solide jaune.

Spectre de R.M.N. ¹H (400 MHz, CDCl₃, δ en ppm) : 0,96 (d, J = 6,5 Hz : 3H) ; 1,01 (d, J = 6,5 Hz : 3H) ; 1,12 (d, J = 6,5 Hz : 3H) ; de 1,65 à 2,05 (mt : 4H) ; 1,77 (s : 3H) ; 2,17 (mt : 1H) ; 2,76 (mt : 1H) ; de 3,30 à 3,45 (mt : 2H) ; 3,99 (d large, J = 17 Hz : 1H) ; de 4,00 à 4,15 (mt : 2H) ; 4,46 (mt : 1H) ; 4,81 (dd, J = 10 et 1,5 Hz : 1H) ; 4,93 (dd, J = 8 et 3 Hz : 1H) ; de 5,50 à 5,65 (mt : 2H) ; de 5,65 à 5,80 (mt : 2H) ; 5,81 (dd, J = 16 5 et 1,5 Hz : 1H) ; 6,11 (d, J = 16 Hz : 1H) ; 6,51 (dd, J = 16,5 et 5 Hz : 1H) ; 7,23 (mt : 1H) ; 8,42 (s : 1H).

La 16,17-déhydro-16-trifluorométhanesulfonyloxy pristinamycine II_{B} peut être préparée de la manière suivante :
A 5 g de pristinamycine II_{B} en solution dans 210 cm³ de dichlorométhane, on ajoute à -70°C, sous atmosphère d'argon, 5 cm³ de diisopropyléthylamine et 2,3 cm³ d'anhydride triflique. Après 2,5 heures d'agitation à -74°C, le mélange réactionnel est versé sur 200 cm³ d'une solution aqueuse saturée de bicarbonate de sodium. La phase organique est décantée puis lavée par 200 cm³ d'une solution aqueuse saturée de chlorure de sodium. La phase organique est décantée, séchée sur sulfate de magnésium, filtrée puis concentrée sous pression réduite à sec (2,7 kPa) pour donner 6,2 g d'un résidu qui est purifié par chromatographie-flash sur silice [éluant : dichlorométhane / acétate d'éthyle (1 / 1 en volumes)]. Après concentration des fractions contenant le produit attendu, on obtient un solide qui est agité dans 60 cm³ d'un mélange pentane / éther éthylique (1 / 1 en volumes) puis filtré et séché sous pression réduite (2,7 kPa) pour donner 2,1 g de 16,17-déhydro-16-trifluorométhanesulfonyloxy pristinamycine II_{B}, sous forme d'un solide blanc fondant vers 140°C avec décomposition.

Spectre de R.M.N. ¹H (400 MHz, CDCl₃, δ en ppm) : 0,96 (d, J = 6,5 Hz : 3H) ; 0,99 (d, J = 6,5 Hz : 3H) ; 1,08 (d, J = 6,5 Hz : 3H) ; de 1,70 à 2,05 (mt : 4H) ; 1,80 (s : 3H) ; 2,17 (mt : 1H) ; 2,76 (mt : 1H) ; 3,13 (dd, J = 15 et 5,5 Hz : 1H) ; 3,46 (mt : 1H) ; 3,60 (dd, J = 15 et 6,5 Hz : 1H) ; 3,72 (mt : 1H) ; 3,96 (mt : 1H) ; 4,38 (mt : 1H) ; 4,73 (dd, J = 10 et 2 Hz : 1H) ; 4,80 (dd, J = 8,5 et 4 Hz : 1H) ; 4,90 (mt : 1H) ; 5,61 (d large, J = 9 Hz : 1H) ; de 5,75 à 5,85 (mt : 1H) ; 5,78 (dd, J = 16 et 1,5 Hz : 1H) ; 6,05 (mt : 1H) ; 6,09 (d, J = 16,5 Hz : 1H) ; 6,52 (dd, J = 16 et 5,5 Hz : 1H) ; 6,56 (s : 1H) ; 8,19 (s : 1H).

### Exemple 2

### (16R)-16-Cyano-16-désoxo pristinamycine II_{B} :

### (16S)-16-Cyano-16-désoxo pristinamycine II_{B} :

Une solution de 2,5 g de 16-cyano-16,17-déhydro-16-désoxo pristinamycine II_{B} dans 375 cm³ d'une solution tampon préalablement préparée par dégazage, sous argon pendant 15 minutes, d'une solution de 21,7 g de tétrafluoroborate de tétraéthylammonium, 26,2 g d'acétate de tétraéthylammonium tétrahydraté et de 5,7 cm³ d'acide acétique, est transvasée sous atmosphère d'argon dans une cuve à électrolyse de 1000 cm³ (potentiostat-galvanostat: 555A Amel; intégrateur: 731 Amel ; millivoltmètre : minisis 6000 Tacussel ; électrode de travail : nappe de mercure d'environ 5 cm², contact par fil de platine plongeant dans le mercure par une tubulure latérale fermée par un rodage sphérique ; électrode de référence (Ag / AgCl) : ECS ; contre électrode : fil de platine enroulé en spirale plongeant dans l'électrolyte séparé du compartiment cathodique par une membrane cationique Nafion 125 non tramé, la membrane est disposée à l'extrémité d'un tube fileté et fixée par un bouchon Téflon fileté, un joint Téflon assure l'étanchéité ; cellule metrohm en verre modifié (volume utile variant de 10 cm³ à 60 cm³ selon les essais)) et dégazée sous argon pendant 15 minutes. On impose une différence de potentiel de -1,5 V (I = 250 mA) pendant 3 heures (consommation de 906 Coulombs). Le mélange réactionnel est prélevé de la cuve à électrolyse puis concentré sous pression réduite à sec (2,7 kPa). Le résidu, auquel on ajoute un autre résidu obtenu d'un essai identique à partir de 2 g de 16-cyano-16,17-déhydro-16-désoxo pristinamycine II_{B}, est repris avec 500 cm³ d'acétate d'éthyle. Cette phase organique est successivement lavée par 200 cm³ d'une solution tampon pH7, 150 cm³ d'eau, 100 cm³ d'une solution tampon pH7 puis séchée sur sulfate de magnésium, filtrée et concentrée sous pression réduite à sec (2,7 kPa) pour donner 4,25 g d'un solide jaune orange auquel on ajoute 2,6 g d'un solide jaune obtenu d'un essai identique (à partir de 2,79 g de 16-cyano-16,17-déhydro-16-désoxo pristinamycine II_{B}). Après purification par chromatographie-flash sur silice [éluant : dichlorométhane / acétonitrile / méthanol (92 / 4 / 4 en volumes)] et concentration des fractions contenant les produits attendus, on obtient 2 solides qui sont agités dans 10 cm³ de diéthyl oxyde puis filtrés et séchés sous pression réduite pour donner respectivement 0,73 g de l'isomère A de la 16-cyano-16-désoxo pristinamycine II_{B}, sous forme d'une poudre jaune pâle fondant vers 130°C (déc.) et 0,725 g de l'isomère B de la 16-cyano-16-désoxo pristinamycine II_{B}, sous forme d'une poudre jaune fondant vers 130°C avec décomposition.

**Isomère A :** Spectre de R.M.N. ¹H (400 MHz, CDCl₃, δ en ppm).

0,96 (d, J = 6,5 Hz : 3H) ; 0,99 (d, J = 6,5 Hz : 3H) ; 1,09 (d, J = 6,5 Hz : 3H) ; de 1,70 à 2,05 (mt : 5H) ; 1,82 (s : 3H) ; 2,13 (mt : 1H) ; 2,22 (d, J = 3,5 Hz : 1H) ; 2,43 (ddd, J = 16 - 10 et 4 Hz : 1H) ; 2,75 (mt : 1H) ; 3,08 (dd, J = 16 et 5 Hz : 1H) ; 3,21 (dd, J = 16 et 6 Hz : 1H) ; 3,30 (mt : 1H) ; 3,49 (ddd, J = 15 - 10 et 4Hz : 1H) ; 3,92 (mt : 2H) ; 4,36 (ddd, J = 15 - 8 et 4 Hz : 1H) ; de 4,70 à 4,85 (mt : 2H) ; 4,96 (mt : 1H) ; 5,55 (d large, J = 9 Hz : 1H) ; 5,68 (ddd, J = 16 - 10 et 4 Hz : 1H) ; 5,78 (dd, J = 16 et 1,5 Hz : 1H) ; 5,94 (dd large, J = 8 et 4 Hz : 1H) ; 6,19 (d large, J = 16 Hz : 1H) ; 6,54 (dd, J = 16 et 5 Hz : 1H) ; 8,14 (s : 1H).

**Isomère B :** Spectre de R.M.N. ¹H (400 MHz, CDCl₃, δ en ppm).

0,96 (d, J = 6,5 Hz : 3H) ; 1,01 (d, J = 6,5 Hz : 3H) ; 1,10 (d, J = 6,5 Hz : 3H) ; 1,61 (ddd, J = 15 - 10 et 4 Hz : 1H) ; de 1,75 à 2,05 (mt : 4H) ; 1,91 (s : 3H) ; 2,09 (d, J = 4 Hz : 1H) ; de 2,05 à 2,30 (mt : 2H) ; 2,77 (mt : 1H) ; 2,92 (dd, J = 16 et 5,5 Hz : 1H) ; de 3,20 à 3,35 (mt : 2H) ; 3,49 (mt : 1H) ; 3,86 (mt : 1H) ; 4,10 (mt : 1H) ; 4,56 (mt : 1H) ; de 4,75 à 4,90 (mt : 1H) ; 4,79 (dd, J = 10 et 1,5 Hz : 1H) ; 4,84 (dd, J = 9 et 4 Hz : 1H) ; 5,36 (d large, J = 9 Hz : 1H) ; 5,77 (ddd, J = 16 - 8,5 et 4 Hz : 1H) ; 5,83 (dd, J = 16 et 1,5 Hz : 1H) ; 6,02 (dd large, J= 9 et 3 Hz : 1H) ; 6,21 (d large, J = 16 Hz : 1H) ; 6,53 (dd, J= 16 et 5 Hz : 1H) ; 8,16 (s : 1H).

La 16-cyano-16,17-déhydro-16-désoxo pristinamycine II_{B} peut être préparée de la manière suivante :
Une solution de 2,3 g de 16,17-déhydro-16-trifluorométhanesulfonyloxy pristinamycine II_{B} (préparée comme décrit dans l'exemple 1), 0,36 g de cyanure de potassium, 0,16 g de palladium tétrakistriphénylphosphine et 0,53 g d'iodure cuivre dans 25 cm³ d'acétonitrile est chauffée au reflux sous atmosphère d'argon. Après 4 heures d'agitation, le mélange réactionnel est filtré sur Célite® et le filtrat est concentré sous pression réduite à sec (2,7 kPa). Le résidu est repris dans 50 cm³ de dichlorométhane et lavé par 2 fois 40 cm³ d'eau. La phase organique est séchée sur sulfate de magnésium, filtrée puis concentrée sous pression réduite à sec (2,7 kPa) pour donner 2 g d'une meringue ocre qui est purifiée par deux chromatographies-flash sur silice successives [éluant : dichlorométhane / acétonitrile / méthanol (96 / 2 / 2 en volumes) puis gradient dichlorométhane / acétonitrile / méthanol (100 / 0 / 0 à 96 / 2 / 2 en volumes)]. Après concentration des fractions contenant le produit attendu, on obtient un solide qui est agité dans 10 cm³ de diéthyl oxyde puis filtré et séché sous pression réduite (2,7 kPa) pour donner 0,19 g de 16-cyano-16,17-déhydro-16-désoxo pristinamycine II_{B} sous forme d'un solide jaune pâle fondant vers 142°C avec décomposition.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,08 (d, J = 6,5 Hz : 3H) ; de 1,70 à 2,05 (mt : 4H) ; 1,78 (d, J = 5 Hz : 1H) ; 1,82 (s : 3H) ; 2,16 (mt : 1H) ; 2,78 (mt : 1H) ; 3,03 (dd, J = 14 et 5 Hz : 1H) ; de 3,40 à 3,50 (mt : 1H) ; 3,50 (dd, J = 14 et 6,5 Hz : 1H) ; 3,72 (mt : 1H) ; 3,96 (mt : 1H) ; 4,36 (ddd, J = 16 - 9 et 4 Hz : 1H) ; 4,72 (dd, J = 10 et 2 Hz : 1H) ; 4,80 (dd, J = 9 et 4,5 Hz : 1H) ; 4,91 (mt : 1H) ; 5,58 (d large, J = 9 Hz : 1H) ; 5,77 (dd, J = 16 et 1,5 Hz : 1H) ; 5,79 (ddd, J = 16 - 9 et 4 Hz : 1H) ; 6,00 (dd large, J = 9 et 5 Hz : 1H) ; 6,10 (d large, J = 16 Hz : 1H) ; 6,51 (dd, J = 16 et 5 Hz : 1H) ; 7,08 (s : 1H) ; 8,26 (s : 1H).

La présente invention concerne également les compositions pharmaceutiques contenant au moins un dérivé de streptogramine selon l'invention, à l'état pur, associé à au moins un dérivé de streptogramine du groupe B, le cas échéant sous forme de sel, et/ou sous forme d'une association avec un ou plusieurs diluants ou adjuvants compatibles et pharmaceutiquement acceptables.

Les compositions selon l'invention peuvent être utilisées par voie orale, parentérale, topique, rectale ou en aérosols.

Comme compositions solides pour administration orale peuvent être utilisés des comprimés, des pilules, des gélules, des poudres ou des granulés. Dans ces compositions, le produit actif selon l'invention, généralement sous forme d'association est mélangé à un ou plusieurs diluants ou adjuvants inertes, tels que saccharose, lactose ou amidon. Ces compositions peuvent comprendre des substances autres que les diluants, par exemple un lubrifiant tel que le stéarate de magnésium ou un enrobage destiné à une libération contrôlée.

Comme compositions liquides pour administration orale, on peut utiliser des solutions pharmaceutiquement acceptables, des suspensions, des émulsions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer le propylèneglycol, un polyéthylèneglycol, des huiles végétales, en particulier l'huile d'olive, des esters organiques injectables, par exemple l'oléate d'éthyle. Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration topique peuvent être par exemple des crèmes, des pommades, des lotions ou des aérosols.

Les compositions par administration rectale sont les suppositoires ou les capsules rectales, qui contiennent outre le principe actif, des excipients tels que le beurre de cacao, des glycérides semi-synthétiques ou des polyéthylèneglycols.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine, les nouveaux dérivés de streptogramine selon l'invention sont particulièrement utiles dans le traitement des infections d'origine bactérienne. Les doses dépendent de l'effet recherché et de la durée du traitement. Le médecin déterminera la posologie qu'il estime la plus appropriée en fonction du traitement, en fonction de l'âge, du poids, du degré de l'infection et des autres facteurs propres au sujet à traiter. Généralement, les doses sont comprises entre 0,5 et 3 g de produit actif en 2 ou 3 administrations par jour, par voie orale ou parentérale pour un adulte.

L'exemple suivant illustre une composition selon l'invention.

### EXEMPLE A

On prépare selon la technique habituelle des comprimés dosés à 250 mg de produit actif, ayant la composition suivante :
- (16*R*)-16-cyano-16-désoxo pristinamycine II_{B} 175 mg
- pristinamycine I_{B} 75 mg
- excipient : amidon, silice hydratée, dextrine, gélatine, stéarate de magnésium : qsp 500 mg

### EXEMPLE B

On prépare selon la technique habituelle des comprimés dosés à 250 mg de produit actif, ayant la composition suivante :
- (16*S*)-16-cyano-16-désoxo pristinamycine II_{B} 175 mg
- pristinamycine I_{B} 75 mg
- excipient : amidon, silice hydratée, dextrine, gélatine, stéarate de magnésium : qsp 500 mg

## Revendications

1. Un dérivé du groupe A des streptogramines de formule générale : 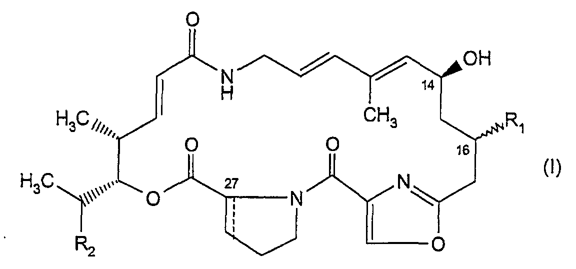 dans laquelle :
- R₁ représente des radicaux cyano ou éthynyle,
- R₂ représente un atome d'hydrogène ou un radical méthyle ou éthyle, et
- la liaison --- représente une liaison simple (stéréochimie 27R) ou une liaison double.

2. Un dérivé de streptogramine selon la revendication 1, **caractérisé en ce que**
- R₁ représente un radical cyano ou éthynyle,
- R₂ représente un radical méthyle, et
- la liaison --- représente une liaison simple (stéréochimie 27R) ou une liaison double.

3. Un procédé de préparation d'un dérivé du groupe A des streptogramines selon la revendication 1, **caractérisé en ce que** l'on opère à partir d'un dérivé sulfonyloxy-16 de formule générale : 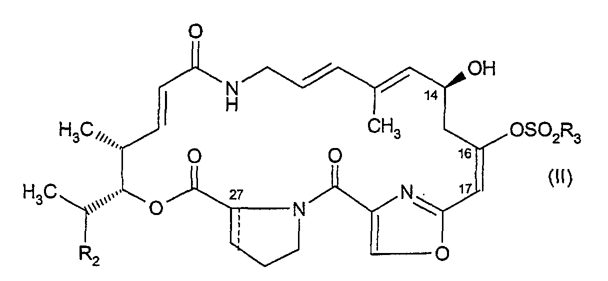 dans laquelle R₂ est défini comme dans la revendication 1, R₃ est un radical alcoyle perfluoré contenant 1 à 10 atomes de carbone et la liaison --- représente une liaison simple (stéréochimie 27R) ou une liaison double, soit par action d'un cyanure alcalin, suivie de la réduction du produit obtenu, soit par carbonylation, suivie de la réduction de la lactone formée en lactol et suivie de l'action du diazométhyl phosphonate de diméthyle ou du 1-diazo-2-oxopropyl phosphonate de diméthyle, puis éventuellement séparation des isomères 16R et 16S obtenus.

4. Un procédé de préparation selon la revendication 2, **caractérisé en ce que** le radical R₃ est un radical trifluorométhyle ou nonafluorobutyle.

5. Un dérivé du groupe A des streptogramines **caractérisé en ce qu'**il répond à la formule générale : 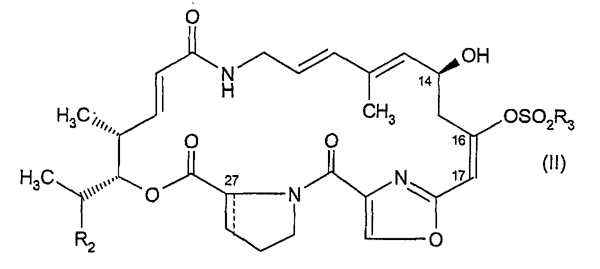 dans laquelle R₂ est défini comme dans la revendication 1, R₃ est un radical alcoyle perfluoré contenant 1 à 10 atomes de carbone et la liaison --- représente une liaison simple (stéréochimie 27R) ou une liaison double.

6. Un dérivé du groupe A des streptogramines selon la revendication 4, **caractérisé en ce que** le radical R₃ est un radical trifluorométhyle ou nonafluorobutyle.

7. Un dérivé du groupe A des streptogramines **caractérisé en ce qu'**il répond à la formule générale : 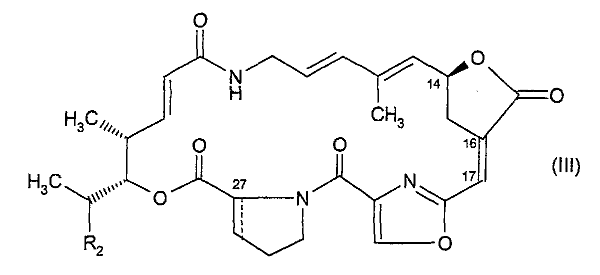 dans laquelle R₂ est défini comme dans la revendication 1 et la liaison --- représente une liaison simple (stéréochimie 27R) ou une liaison double.

8. Composition pharmaceutique comprenant un dérivé de streptogramine du groupe A selon la revendication 1, à l'état pur ou sous forme d'association avec au moins un dérivé de streptogramine du groupe B, et/ou sous forme d'association avec un ou plusieurs diluants ou adjuvants compatibles et pharmaceutiquement acceptables.

9. Composition pharmaceutique selon la revendication 8, **caractérisée en ce que** le dérivé de la streptogramine du groupe B est choisi parmi les composantes naturelles : pristinamycine IA, pristinamycine IB, pristinamycine IC, pristinamycine ID, pristinamycine IE, pristinamycine IF, pristinamycine IG, virginiamycine S1, S3 ou S4, vernamycine B ou C, étamycine ou parmi des dérivés d'hémisynthèse de formule générale : 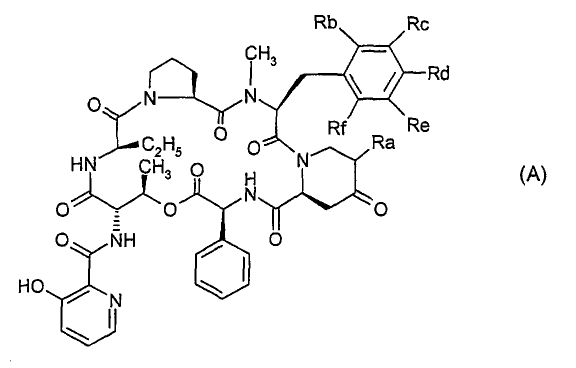 dans laquelle,
1) Rb, Rc, Re et Rf sont des atomes d'hydrogène, Rd est un atome d'hydrogène ou un radical diméthylamino, et Ra est un radical de structure -CH₂R' a pour lequel R'a est pyrrolidinyl-3thio, pipéridyl-3(ou -4)thio pouvant être substitués par alcoyle, alcoylthio substitué par 1 ou 2 hydroxysulfonyle, alcoylamino, dialcoylamino (lui même éventuellement substitué par mercapto ou dialcoylamino), ou substitué par 1 ou 2 cycles pipérazine éventuellement substitué, morpholino, thiomorpholino, pipéridino, pyrrolidinyle-1, pipéridyle-2,-3 ou -4, ou pyrrolidinyle-2 ou -3 (pouvant être substitués par alcoyle), ou bien Ra est un radical de structure =CHR'a pour lequel R'a est pyrrolidinyl-3amino, pipéridyl-3(ou -4)amino, pyrrolidinyl-3oxy, pipéridyl-3(ou -4)oxy, pyrrolidinyl-3thio, pipéridyl-3(ou -4)thio pouvant être substitués par alcoyle, ou R'a est alcoylamino, alcoyloxy ou alcoylthio substitués par 1 ou 2 hydroxysulfonyle, alcoylamino, dialcoylamino (lui même éventuellement substitué par dialcoylamino), ou par trialcoylammonio, imidazolyl-4 ou -5, ou par 1 ou 2 cycles pipérazine éventuellement substitué, morpholino, thiomorpholino, pipéridino, pyrrolidinyle-1, pipéridyle-2,-3 ou -4, ou pyrrolidinyle-2 ou -3 (pouvant être substitués par alcoyle), ou Ra est un radical quinuclidinyl-3(ou -4)thiométhyle ou bien
2) Ra est un atome d'hydrogène et
a) soit Rb, Re et Rf sont des atomes d'hydrogène, Rd est un radical -NHCH₃ ou -N(CH₃)₂ et Rc est un atome de chlore ou de brome, ou représente un radical alcényle contenant 3 à 5 atomes de carbone [si Rd est -N(CH₃)₂],
b) soit Rb, Rd, Re et Rf représentent un atome d'hydrogène et Rc est un halogène, ou un radical aminomonoalkyle, aminodialkyle, alcoyloxy, trifluorométhyloxy, thioalcoyle,alcoyle en C₁ à C₃ ou trihalogénométhyle
c) soit Rb, Rc, Re et Rf représentent un atome d'hydrogène et Rd est un halogène, ou un radical éthylamino, diéthylamino ou méthyléthylamino, alcoyloxy ou trifluorométhyloxy, thioalkyle, alcoyle en C₁ à C₆, aryle ou trihalogénométhyle
d) soit Rb, Re et Rf représentent un atome d'hydrogène et Rc est halogène ou un radical aminomonoalkyle ou aminodialkyle, alcoyloxy ou trifluorométhyloxy, thioalkyle, alcoyle en C₁ à C₃, et Rd est halogène ou un radical amino, aminomonoalkyle ou aminodialkyle, alcoyloxy ou trifluorométhyloxy, thioalkyle, alcoyle en C₁ à C₆ ou trihalogénométhyle,
e) soit Rc, Re et Rf représentent un atome d'hydrogène et Rb et Rd représentent un radical méthyle ;
ou encore parmi les dérivés d'hémisynthèse de formule générale : 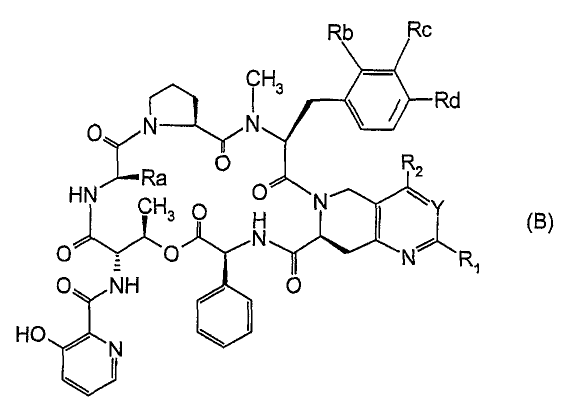 dans laquelle
Y est un atome d'azote ou un radical =CR₃-,
R₁ est un atome d'hydrogène, un radical alcoyle (1 à 8 carbones), alcényle (2 à 8 carbones), cycloalcoyle (3 à 8 carbones), hétérocyclyle saturé ou insaturé (3 à 8 chaînons), phényle, phényle substitué [par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino] ou un radical NR'R", R' et R" identiques ou différents pouvant être des atomes d'hydrogène ou des radicaux alcoyle (1 à 3 carbones), ou pouvant former ensemble avec l'atome d'azote auquel ils sont attachés, un hétérocycle de 3 à 8 chaînons contenant éventuellement un autre hétéroatome choisi parmi l'oxygène le soufre ou l'azote éventuellement substitué [par un radical alcoyle, alcényle (2 à 8 carbones), cycloalcoyle (3 à 6 carbones), hétérocyclyle saturé ou insaturé (4 à 6 chaînons), benzyle, phényle ou phényle substitué tel que défini ci-dessus pour la définition de R₁]
ou bien lorsque Y est un radical =CR₃-, R₁ peut être aussi halogénométhyle, hydroxyméthyle, alcoyloxyméthyle, alcoylthiométhyle dont la partie alcoyle est éventuellement substituée par NR'R", alcoylsulfinylméthyle, alcoylsulfonylméthyle, acyloxyméthyle, benzoyloxyméthyle, cyclopropylaminométhyle ou -(CH₂)ₙNR'R" (n étant un entier de 1 à 4 et R' et R" étant définis comme ci-dessus), ou bien si R₃ est un atome d'hydrogène, R₁ peut être aussi formyle, carboxy, alcoyloxycarbonyle, ou -CONR'R" pour lequel R' et R" sont définis comme ci-dessus,
ou bien lorsque Y est un atome d'azote, R₁ peut être aussi un radical -XR° pour lequel X est un atome d'oxygène ou de soufre, un radical sulfinyle ou sulfonyle,
ou un radical NH et R° est un radical alcoyle (1 à 8 carbones), cycloalcoyle (3 à 6 carbones), hétérocyclyle saturé ou insaturé (3 à 8 chaînons), hétérocyclylméthyle (3 à 8 chaînons) dont la partie hétérocyclyle est attachée au radical méthyle par un atome de carbone, phényle, phényle substitué [par un ou plusieurs atomes d'halogène ou radicaux hydroxy, alcoyle, alcoyloxy, alcoylthio, alcoylsulfmyle, alcoylsulfonyle, amino, alcoylamino ou dialcoylamino] ou un radical -(CH₂)ₙNR'R" pour lequel R' et R" sont définis comme ci-dessus et n est un entier de 2 à 4, ou bien, si X représente NH, R° peut aussi représenter l'atome d'hydrogène,
R₂ est un atome d'hydrogène ou un radical alcoyle (1 à 3 carbones),
R₃ est un atome d'hydrogène ou un radical alcoyle, carboxy, alcoyloxycarbonyle ou carbamoyle de structure -CO-NR'R" dans laquelle R' et R" sont définis comme précédemment,
Ra est un radical méthyle ou éthyle, et
Rb, Rc et Rd ont les définitions ci-après :
1) Rb et Rc sont des atomes d'hydrogène et Rd est un atome d'hydrogène ou un radical méthylamino ou diméthylamino,
2) Rb est un atome d'hydrogène, Rc est un atome d'hydrogène, de chlore ou de brome, ou représente un radical alcényle (3 à 5C), et Rd est un radical -NMe-R"' pour lequel R"' représente un radical alcoyle, hydroxyalcoyle (2 à 4C), ou alcényle (2 à 8C) éventuellement substitué par phényle, cycloalcoyl(3 à 6C)méthyle, benzyle, benzyle substitué [par un ou plusieurs atomes d'halogène ou radicaux hydroxy, alcoyle, alcoyloxy, alcoylthio, alcoylsulfmyle, alcoylsulfonyle, amino, alcoylamino ou dialcoylamino], hétérocyclylméthyle ou hétérocyclyléthyle dont la partie hétérocyclyle est saturée ou insaturée et contient 5 à 6 chaînons et 1 ou 2 hétéroatomes choisis parmi le soufre, l'oxygène ou l'azote éventuellement substitué [par un radical alcoyle, alcényle (2 à 8 carbones), cycloalcoyle (3 à 6 carbones), hétérocyclyle saturé ou insaturé (4 à 6 chaînons), phényle, phényle substitué tel que défini ci-avant pour la définition de R₁ ou benzyle], ou bien R"' représente un radical cyanométhyle, ou -CH₂CORe pour lequel soit Re est -OR'e, R'e étant hydrogène, alcoyle (1 à 6 carbones), alcényle (2 à 6 carbones), benzyle ou hétérocyclylméthyle dont la partie hétérocyclyle contient 5 à 6 chaînons et 1 ou 2 hétéroatomes choisis parmi le soufre, l'oxygène ou l'azote soit Re est un radical alcoylamino, alcoyl méthyl amino, hétérocyclylamino ou hétérocyclyl méthyl amino dont la partie hétérocyclyle est saturée et contient 5 à 6 chaînons et 1 ou 2 hétéroatomes choisis parmi le soufre, l'oxygène ou l'azote éventuellement substitué par un radical alcoyle, benzyle ou alcoyloxycarbonyle,
3) Rb est un atome d'hydrogène, Rd est un radical -NHCH₃ ou -N(CH₃)₂ et Rc est un atome de chlore ou de brome; ou représente un radical alcényle (3 à 5C), [si Rd est -N(CH₃)₂],
4) Rb et Rd sont des atomes d'hydrogène et Rc est un atome d'halogène, ou un radical alcoylamino ou dialcoylamino, alcoyloxy, trifluorométhoxy, thioalcoyle, alcoyle (1 à 6C) ou trihalogénométhyle,
5) Rb et Rc sont des atomes d'hydrogène et Rd est un atome d'halogène, ou un radical éthylamino, diéthylamino ou méthyl éthyl amino, alcoyloxy ou trifluorométhoxy, alcoylthio, alcoylsulfmyle, alcoylsulfonyle, alcoyle (1 à 6C), phényle ou trihalogénométhyle,
6) Rb est un atome d'hydrogène et Rc est un atome d'halogène ou un radical alcoylamino ou dialcoylamino, alcoyloxy ou trifluorométhoxy, thioalcoyle, alcoyle (1 à 3C), et Rd est un atome d'halogène ou un radical amino, alcoylamino ou dialcoylamino, alcoyloxy ou trifluorométhoxy, thioalcoyle, alcoyle (1 à 6C) ou trihalogénométhyle,
7) Rc est un atome d'hydrogène et Rb et Rd représentent un radical méthyle,
ainsi que leurs sels,
ou encore parmi les dérivés d'hémisynthèse de formule générale : 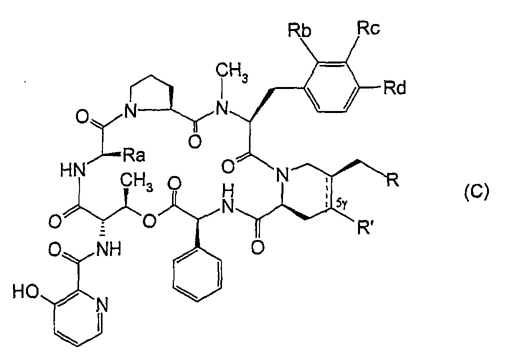 dans laquelle R représente un radical -NR₁R₂ ou -SR₃ [pour lequel R₁ et R₂ identiques ou différents représentent H, alcoyle (1 à 8C) éventuellement substitué par OH, alcényle (3 à 8C), cycloalcoyle (3 à 8C), alcoyloxy (1 à 8C), dialcoylamino, phénylalcoyle éventuellement substitué [par un ou plusieurs halogène ou alcoyle, hydroxyalcoyle, alcoyloxy ou dialcoylamino], hétérocyclylalcoyle saturé ou insaturé (3 à 8 chaînons) contenant 1 ou plusieurs hétéroatomes choisis parmi N, S ou O, ou bien R₁ et R₂ forment ensemble avec l'atome d'azote, un hétérocycle mono ou polycyclique, saturé, partiellement saturé ou insaturé (3 à 12 chaînons), contenant éventuellement un autre hétéroatome choisi parmi N, S ou O, et éventuellement substitué [par un ou plusieurs OH, alcoyle, phényle éventuellement substitué par un atome d'halogène, phénylalcoyle, phénylalcényle (alcényle contenant 2 à 4C), hydroxyalcoyle, acyle, alcoyloxycarbonyle, ou hétérocyclyle ou hétérocyclylcarbonyle dont la partie hétérocyclyle est saturée ou insaturée (4 à 6 chaînons) et contient 1 ou plusieurs hétéroatomes choisis parmi N, S ou O,], R₃ est alcoyle (1 à 8C) ou cycloalcoyle (3 à 8C) substitués par -NR₁R₂ pour lequel R₁ et R₂ identiques ou différents sont H ou alcoyle ou forment ensemble avec l'atome d'azote auquel ils sont attachés, un hétérocycle tel que défini ci-dessus,
ou bien R₃ est hétérocyclyle ou hétérocyclylméthyle saturé ou insaturé (3 à 7 chaînons) et éventuellement un autre hétéroatome choisi parmi l'oxygène le soufre ou l'azote et éventuellement substitué par un radical alcoyle ; 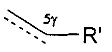 représente le reste d'un cycle insaturé non substitué en 5γ : 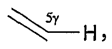 ou le reste d'un cycle saturé substitué en 5γ par un radical fluoro : 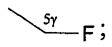 Ra est un radical Me ou Et, et Rb, Rc et Rd sont définis ci-après :
1) Rb et Rc sont H et Rd est H ou un radical MeNH ou NMe₂,
2) Rb est H, Rc est H, Cl ou Br, ou alcényle (3 à 5C), et Rd est -NMe-R"', R'" étant alcoyle, hydroxyalcoyle (2 à 4C), ou alcényle (2 à 8C), phénylalcényle, cycloalcoyl(3 à 6C)méthyle, benzyle, benzyle substitué, hétérocyclylméthyle, hétérocyclyléthyle, ou bien R"' est -CH₂CN, -CH₂COOH ou -CORe ou -CH₂CORe pour lesquels soit Re est -OR'e, soit Re est alcoylamino, alcoyl méthyl amino, hétérocyclylamino ou hétérocyclyl méthyl amino,
3) Rb est H, Rd est un radical -NHCH₃ ou -N(CH₃)₂ et Rc est Cl ou Br, ou alcényle (3 à 5C), [si Rd est -N(CH₃)₂],
4) Rb et Rd sont H et Rc est halogène, ou alcoylamino ou dialcoylamino, alcoyloxy, trifluorométhoxy, thioalcoyle, alcoyle (1 à 6C) ou trihalogénométhyle,
5) Rb et Rc sont H et Rd est halogène, ou éthylamino, diéthylaxnino ou méthyl éthyl amino, alcoyloxy ou trifluorométhoxy, alcoylthio, alcoylsulfmyle, alcoylsulfonyle, alcoyle (1 à 6C), phényle ou trihalogénométhyle,
6) Rb est H et Rc est halogène ou alcoylamino ou dialcoylamino, alcoyloxy ou trifluorométhoxy, thioalcoyle, alcoyle (1 à 3C), et Rd est halogène ou un radical amino, alcoylamino ou dialcoylamino, alcoyloxy ou trifluorométhoxy, thioalcoyle, alcoyle (1 à 6C) ou trihalogénométhyle
7) Rc est H et Rb et Rd sont CH₃
ainsi que ses sels lorsqu'ils existent.

## Claims

1. Group A streptogramin derivative of general formula: 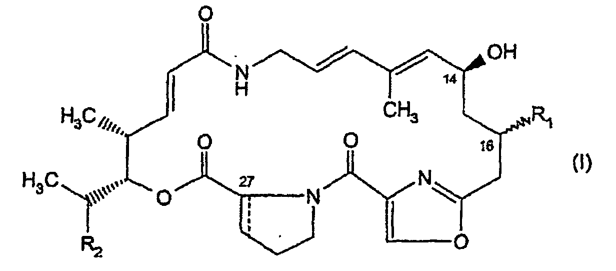 in which:
- R₁ represents cyano or ethynyl radicals,
- R₂ represents a hydrogen atom or a methyl or ethyl radical, and
- the bond --- represents a single bond (27R stereo chemistry) or a double bond.

2. Streptogramin derivative according to Claim 1, **characterized in that**
- R₁ represents a cyano or ethynyl radical,
- R₂ represents a methyl radical, and
- the bond --- represents a single bond (27R stereochemistry) or a double bond.

3. Process for preparing a group A streptogramin derivative according to Claim 1, **characterized in that** the process is performed using a 16-sulphonyloxy derivative of general formula: 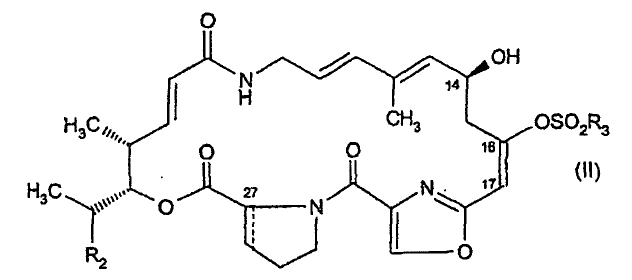 in which R₂ is defined as in Claim 1, R₃ is a perfluoroalkyl radical containing 1 to 10 carbon atoms and the bond --- represents a single bond (27R stereochemistry) or a double bond, either by the action of an alkali metal cyanide, followed by reduction of the product obtained, or by carbonylation, followed by reduction of the lactone formed to a lactol and followed by the action of dimethyl diazomethylphosphonate or dimethyl 1-diazo-2-oxopropylphosphonate and then, optionally, separation of the 16R and 16S isomers obtained.

4. Preparation process according to Claim 2, **characterized in that** the radical R₃ is a trifluoromethyl or nonafluorobutyl radical.

5. Group A streptogramin derivative, **characterized in that** it corresponds to the general formula: 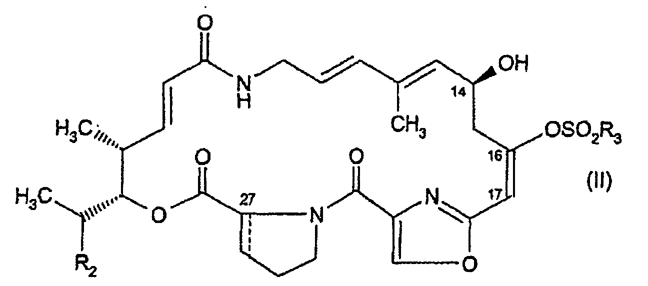 in which R₂ is defined as in Claim 1, R₃ is a perfluoroalkyl radical containing 1 to 10 carbon atoms and the bond --- represents a single bond (27R stereochemistry) or a double bond.

6. Group A streptogramin derivative according to Claim 4, **characterized in that** the radical R₃ is a trifluoromethyl or nonafluorobutyl radical.

7. Group A streptogramin derivative, **characterized in that** it corresponds to the general formula: 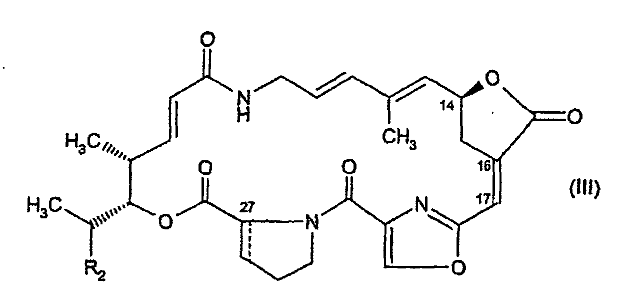 in which R₂ is defined as in Claim 1 and the bond --- represents a single bond (27R stereochemistry) or a double bond.

8. Pharmaceutical composition comprising a group A streptogramin derivative according to Claim 1, in pure form or in the form of a combination with at least one group B streptogramin derivative, and/or in the form of a combination with one or more compatible and pharmaceutically acceptable diluents or adjuvants.

9. Pharmaceutical composition according to Claim 8, **characterized in that** the group B streptogramin derivative is chosen from the following natural components: pristinamycin IA, pristinamycin IB, pristinamycin IC, pristinamycin ID, pristinamycin IE, pristinamycin IF, pristinamycin IG, virginiamycin S1, S3 or S4, vernamycin B or C, etamycin or from semisynthetic derivatives of general formula: 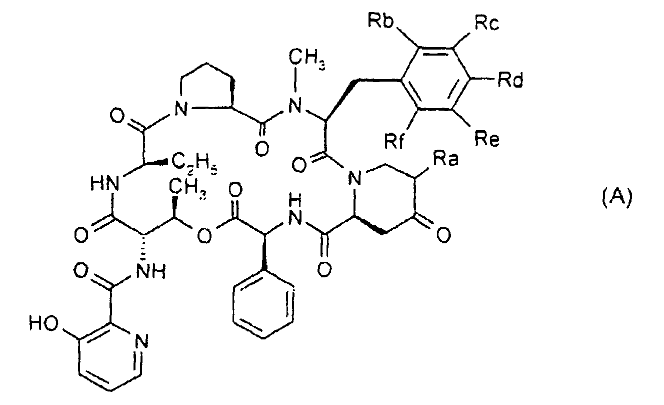 in which:
1) Rb, Rc, Re and Rf are hydrogen atoms, Rd is a hydrogen atom or a dimethylamino radical, and Ra is a radical of structure -CH₂R'a for which R'a is pyrrolidinyl-3-thio, piperidyl-3- (or -4-)thio which may be substituted with alkyl, alkylthio substituted with 1 or 2 hydroxysulphonyl, alkylamino or dialkylamino (which itself may optionally be substituted with mercapto or dialkylamino), or substituted with 1 or 2 piperazine rings, optionally substituted, morpholino, thiomorpholino, piperidino, 1-pyrrolidinyl, 2-, 3- or 4-piperidyl or 2- or 3-pyrrolidinyl rings (which may be substituted with alkyl), or alternatively Ra is a radical of structure =CHR'a for which R'a is pyrrolidinyl-3-amino, piperidyl-3- (or -4-)amino, pyrrolidinyl-3-oxy, piperidyl-3- (or -4-)oxy, pyrrolidinyl-3-thio, piperidyl-3- (or -4-)thio which may be substituted with alkyl, or R'a is alkylamino, alkyloxy or alkylthio which is substituted with 1 or 2 hydroxysulphonyl, alkylamino or dialkylamino (which is itself optionally substituted with dialkylamino), or with trialkylammonio, 4- or 5-imidazolyl, or with 1 or 2 piperazine rings, optionally substituted, morpholino, thiomorpholino, piperidino, 1-pyrrolidinyl, 2-, 3- or 4-piperidyl, or 2- or 3-pyrrolidinyl rings (which may be substituted with alkyl), or
Ra is a quinuclidinyl-3- (or -4-)thiomethyl radical, or alternatively
2) Ra is a hydrogen atom and
a) either Rb, Re and Rf are hydrogen atoms, Rd is an -NHCH₃ or -N(CH₃)₂ radical and Rc is a chlorine or bromine atom, or represents an alkenyl radical containing 3 to 5 carbon atoms [if Rd is -N(CH₃)₂],
b) or Rb, Rd, Re and Rf represent a hydrogen atom and Rc is a halogen or an aminomonoalkyl, aminodialkyl, alkyloxy, trifluoromethyloxy, thioalkyl, C₁ to C₃ alkyl or trihalomethyl radical,
c) or Rb, Rc, Re and Rf represent a hydrogen atom and Rd is a halogen or an ethylamino, diethylamino or methylethylamino, alkyloxy or trifluoromethyloxy, thioalkyl, C₁ to C₆ alkyl, aryl or trihalomethyl radical,
d) or Rb, Re and Rf represent a hydrogen atom and Rc is halogen or an aminomonoalkyl or aminodialkyl, alkyloxy or trifluoromethyloxy, thioalkyl or C₁ to C₃ alkyl radical, and Rd is halogen or an amino, aminomonoalkyl or aminodialkyl, alkyloxy or trifluoromethyloxy, thioalkyl, C₁ to C₆ alkyl or trihalomethyl radical,
e) or Rc, Re and Rf represent a hydrogen atom and Rb and Rd represent a methyl radical;
or alternatively from the semisynthetic derivatives of general formula: 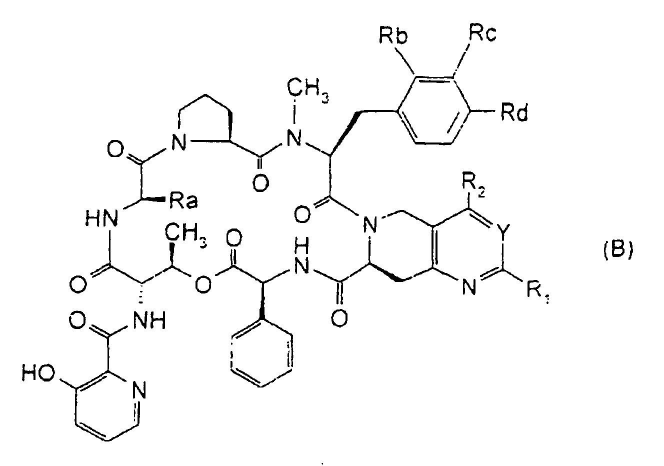 in which:
Y is a nitrogen atom or a radical =CR₃-,
R₁ is a hydrogen atom, an alkyl radical (1 to 8 carbons), alkenyl radical (2 to 8 carbons), cycloalkyl radical (3 to 8 carbons), saturated or unsaturated heterocyclyl radical (3- to 8-membered), phenyl radical, substituted phenyl radical [substituted with one or more halogen atoms or hydroxyl, alkyl, alkyloxy, alkylthio, alkylsulphinyl, alkylsulphonyl, amino, alkylamino or dialkylamino radicals] or a radical NR'R", R' and R", which may be identical or different, possibly being hydrogen atoms or alkyl radicals (1 to 3 carbons) or possibly forming, together with the nitrogen atom to which they are attached, a 3- to 8-membered heterocycle optionally containing another hetero atom chosen from oxygen, sulphur and nitrogen which is optionally substituted [with an alkyl radical, alkenyl radical (2 to 8 carbons), cycloalkyl radical (3 to 6 carbons), saturated or unsaturated heterocyclyl radical (4- to 6-membered), benzyl radical, phenyl radical or substituted phenyl radical as defined above for the definition of R₁],
or alternatively, when Y is a radical =CR₃-, R₁ may also be halomethyl, hydroxymethyl, alkyloxymethyl, alkylthiomethyl in which the alkyl portion is optionally substituted with NR'R'', alkylsulphinylmethyl, alkylsulphonylmethyl, acyloxymethyl, benzoyloxymethyl, cyclopropylaminomethyl or - (CH₂) ₙNR' R" (n being an integer from 1 to 4 and R' and R" being defined as above), or alternatively, if R₃ is a hydrogen atom, R₁ may also be formyl, carboxyl, alkyloxycarbonyl or -CONR'R" for which R' and R" are defined as above,
or alternatively, when Y is a nitrogen atom, R₁ may also be a radical -XR° for which X is an oxygen or sulphur atom, a sulphinyl or sulphonyl radical, or an NH radical and R° is an alkyl radical (1 to 8 carbons), cycloalkyl radical (3 to 6 carbons), saturated or unsaturated heterocyclyl radical (3- to 8-membered), heterocyclylmethyl radical (3- to 8-membered) in which the heterocyclyl portion is attached to the methyl radical via a carbon atom, phenyl radical, substituted phenyl radical [substituted with one or more halogen atoms or hydroxyl, alkyl, alkyloxy, alkylthio, alkylsulphinyl, alkylsulphonyl, amino, alkylamino or dialkylamino radicals] or a radical -(CH₂)ₙNR'R" for which R' and R" are defined as above and n is an integer from 2 to 4, or alternatively, if X represents NH, R° may also represent a hydrogen atom,
R₂ is a hydrogen atom or an alkyl radical (1 to 3 carbons),
R₃ is a hydrogen atom or an alkyl, carboxyl, alkyloxycarbonyl or carbamoyl radical of structure -CO-NR'R" in which R' and R" are defined as above,
Ra is a methyl or ethyl radical, and
Rb, Rc and Rd have the definitions below:
1) Rb and Rc are hydrogen atoms and Rd is a hydrogen atom or a methylamino or dimethylamino radical,
2) Rb is a hydrogen atom, Rc is a hydrogen, chlorine or bromine atom, or represents an alkenyl radical (3 to 5C), and Rd is a radical -NMe-R"' for which R"' represents an alkyl radical, hydroxyalkyl radical (2 to 4C) or alkenyl radical (2 to 8C) optionally substituted with phenyl, cycloalkyl(3 to 6C)methyl radical, benzyl radical, substituted benzyl radical [substituted with one or more halogen atoms or hydroxyl, alkyl, alkyloxy, alkylthio, alkylsulphinyl, alkylsulphonyl, amino, alkylamino or dialkylamino radicals], heterocyclylmethyl radical or heterocyclylethyl radical in which the heterocyclyl portion is saturated or unsaturated and 5- or 6-membered and contains 1 or 2 hetero atoms chosen from sulphur, oxygen and nitrogen which is optionally substituted [with an alkyl radical, alkenyl radical (2 to 8 carbons), cycloalkyl radical (3 to 6 carbons), saturated or unsaturated heterocyclyl radical (4- to 6-membered), phenyl radical, substituted phenyl radical as defined above for the definition of R₁, or benzyl radical], or alternatively R''' represents a cyanomethyl radical or a radical -CH₂CORe for which either Re is -OR'e, R'e being hydrogen, alkyl (1 to 6 carbons), alkenyl (2 to 6 carbons), benzyl or heterocyclylmethyl in which the heterocyclyl portion is 5- or 6-membered and contains 1 or 2 hetero atoms chosen from sulphur, oxygen and nitrogen, or Re is an alkylamino, alkylmethylamino, heterocyclylamino or heterocyclylmethylamino radical in which the heterocyclyl portion is saturated and 5- or 6-membered and contains 1 or 2 hetero atoms chosen from sulphur, oxygen and nitrogen which is optionally substituted with an alkyl, benzyl or alkyloxycarbonyl radical,
3) Rb is a hydrogen atom, Rd is an -NHCH₃ or -N (CH₃)₂ radical and Rc is a chlorine or bromine atom or represents an alkenyl radical (3 to 5C) [if Rd is -N(CH₃)₂],
4) Rb and Rd are hydrogen atoms and Rc is a halogen atom or an alkylamino or dialkylamino, alkyloxy, trifluoromethoxy, thioalkyl, alkyl (1 to 6C) or trihalomethyl radical,
5) Rb and Rc are hydrogen atoms and Rd is a halogen atom or an ethylamino, diethylamino or methylethylamino, alkyloxy or trifluoromethoxy, alkylthio, alkylsulphinyl, alkylsulphonyl, alkyl (1 to 6C), phenyl or trihalomethyl radical,
6) Rb is a hydrogen atom and Rc is a halogen atom or an alkylamino or dialkylamino, alkyloxy or trifluoromethoxy, thioalkyl or alkyl (1 to 3C) radical and Rd is a halogen atom or an amino, alkylamino or dialkylamino, alkyloxy or trifluoromethoxy, thioalkyl, alkyl (1 to 6C) or trihalomethyl radical,
7) Rc is a hydrogen atom and Rb and Rd represent a methyl radical,
as well as the salts thereof,
or alternatively from the semisynthetic derivatives of general formula: 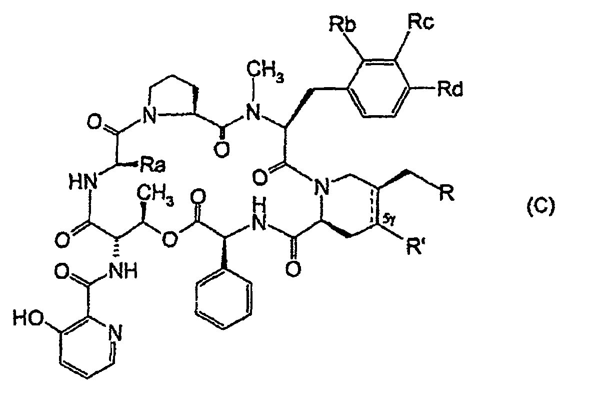 in which R represents a radical -NR₁R₂ or -SR₃ [for which R₁ and R₂, which may be identical or different, represent H, alkyl (1 to 8C) optionally substituted with OH, alkenyl (3 to 8C), cycloalkyl (3 to 8C), alkyloxy (1 to 8C), dialkylamino, phenylalkyl which is optionally substituted [with one or more halogen or alkyl, hydroxyalkyl, alkyloxy or dialkylamino], saturated or unsaturated heterocyclylalkyl (3- to 8-membered) containing one or more hetero atoms chosen from N, S and O, or alternatively R₁ and R₂ form, together with the nitrogen atom, a saturated, partially saturated or unsaturated mono- or polycyclic heterocycle (3- to 12-membered) optionally containing another hetero atom chosen from N, S and O, and optionally substituted [with one or more OH, alkyl, phenyl which is optionally substituted with a halogen atom, phenylalkyl, phenylalkenyl (alkenyl containing 2 to 4C), hydroxyalkyl, acyl, alkyloxycarbonyl or heterocyclyl or heterocyclylcarbonyl in which the heterocyclyl portion is saturated or unsaturated (4- to 6-membered) and contains one or more hetero atoms chosen from N, S and O], R₃ is alkyl (1 to 8C) or cycloalkyl (3 to 8C) substituted with -NR₁R₂ for which R₁ and R₂, which may be identical or different, are H or alkyl or form, together with the nitrogen atom to which they are attached, a heterocycle as defined above, or alternatively R₃ is saturated or unsaturated heterocyclyl or heterocyclylmethyl (3- to 7-membered) optionally containing another hetero atom chosen from oxygen, sulphur and nitrogen and optionally substituted with an alkyl radical; 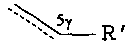 represents the residue of an unsaturated ring which is not substituted in the 5γ position: 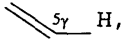 or the residue of a saturated ring which is substituted
in the 5γ position with a fluoro radical: 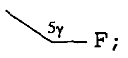 Ra is an Me or Et radical and Rb, Rc and Rd are defined below:
1) Rb and Rc are H and Rd is H or an MeNH or NMe₂ radical,
2) Rb is H, Rc is H, Cl or Br, or alkenyl (3 to 5C), and Rd is -NMe-R"', R"' being alkyl, hydroxyalkyl (2 to 4C) or alkenyl (2 to 8C), phenylalkenyl, cycloalkyl(3 to 6C)methyl, benzyl, substituted benzyl, heterocyclylmethyl or heterocyclylethyl, or alternatively R"' is -CH₂CN, -CH₂COOH or -CORe or -CH₂CORe for which either Re is -OR'e or Re is alkylamino, alkylmethylamino, heterocyclylamino or heterocyclylmethylamino,
3) Rb is H, Rd is an -NHCH₃ or -N (CH₃) ₂ radical and Rc is Cl or Br or alkenyl (3 to 5C) [if Rd is -N(CH₃)₂] ,
4) Rb and Rd are H and Rc is halogen or alkylamino or dialkylamino, alkyloxy, trifluoromethoxy, thioalkyl, alkyl (1 to 6C) or trihalomethyl,
5) Rb and Rc are H and Rd is halogen or ethylamino, diethylamino or methylethylamino, alkyloxy or trifluoromethoxy, alkylthio, alkylsulphinyl, alkylsulphonyl, alkyl (1 to 6C), phenyl or trihalomethyl,
6) Rb is H and Rc is halogen or alkylamino or dialkylamino, alkyloxy or trifluoromethoxy, thioalkyl or alkyl (1 to 3C) and Rd is halogen or an amino, alkylamino or dialkylamino, alkyloxy or trifluoromethoxy, thioalkyl, alkyl (1 to 6C) or trihalomethyl radical,
7) Rc is H and Rb and Rd are CH₃,
as well as the salts thereof, when they exist.

## Patentansprüche

1. Derivat der Gruppe A der Streptogramine der allgemeinen Formel (I) 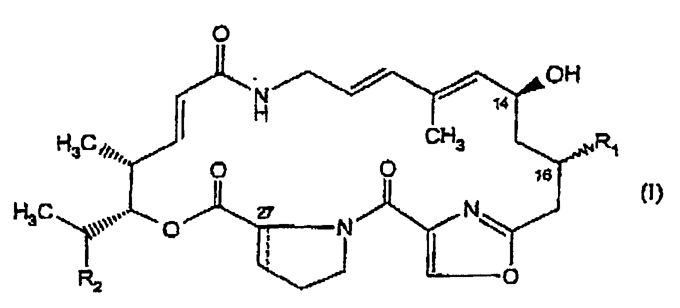 in der
- R₁ Reste Cyano oder Ethinyl darstellt,
- R₂ ein Wasserstoffatom oder einen Rest Methyl oder Ethyl darstellt, und
- die Bindung ---- eine einfache Bindung (Stereochemie 27R) oder eine Doppelbindung darstellt.

2. Streptogramin-Derivat nach Anspruch 1, **dadurch gekennzeichnet, daß**
- R₁ einen Rest Cyano oder Ethinyl darstellt,
- R₂ einen Rest Methyl darstellt, und
- die Bindung ---- eine einfache Bindung (Stereochemie 27R) oder eine Doppelbindung darstellt.

3. Verfahren zur Herstellung eines Derivates der Gruppe A der Streptogramine nach Anspruch 1, **dadurch gekennzeichnet, daß** man ausgehend von einem 16-Sulfonyloxy-Derivat der allgemeinen Formel (II) verfährt: 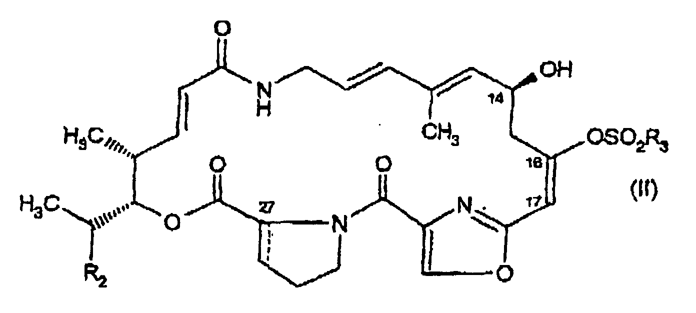 in der R₂ wie in Anspruch 1 definiert ist, R₃ einen perfluorierten Rest Alkyl mit 1 bis 10 Kohlenstoffatomen darstellt und die Bindung ---- eine einfache Bindung (Stereochemie 27R) oder eine Doppelbindung darstellt,
entweder durch Einwirkung eines Alkalicyanids, gefolgt von der Reduktion des erhaltenen Produktes,
oder durch Carbonylierung, gefolgt von der Reduktion des gebildeten Lactons zum Lactol und gefolgt von der Einwirkung von Dimethyl-diazomethyl-phosphonat oder Dimethyl-l-diazo-2-oxopropylphosphonat, und anschließend gegebenenfalls Trennung der erhaltenen Isomeren 16R und 16S.

4. Verfahren zur Herstellung nach Anspruch 2, **dadurch gekennzeichnet, daß** der Rest R₃ ein Rest Trifluormethyl oder Nonafluorbutyl ist.

5. Derivat der Gruppe A der Streptogramine, **dadurch gekennzeichnet, daß** es der allgemeinen Formel (II) entspricht: 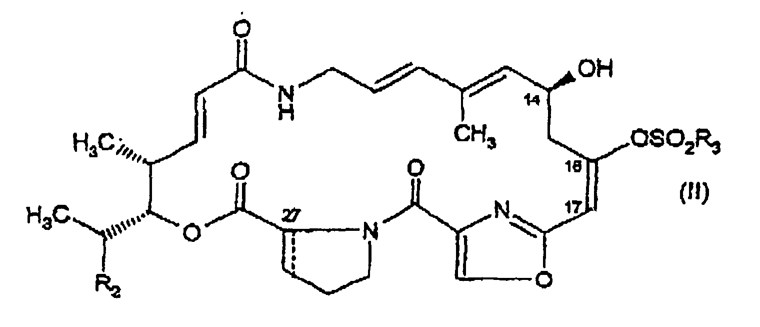 in der R₂ wie in Anspruch 1 definiert ist, R₃ einen perfluorierten Rest Alkyl mit 1 bis 10 Kohlenstoffatomen darstellt und die Bindung ---- eine einfache Bindung (Stereochemie 27R) oder eine Doppelbindung darstellt.

6. Derivat der Gruppe A der Streptogramine nach Anspruch 4, **dadurch gekennzeichnet, daß** der Rest R₃ ein Rest Trifluormethyl oder Nonafluorbutyl ist.

7. Derivat der Gruppe A der Streptogramine, **dadurch gekennzeichnet, daß** es der allgemeinen Formel (III) entspricht: 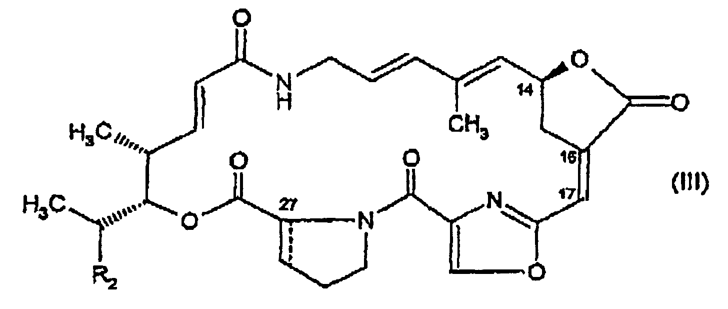 in der R₂ wie in Anspruch 1 definiert ist und die Bindung ---- eine einfache Bindung (Stereochemie 27R) oder eine Doppelbindung darstellt.

8. Pharmazeutische Zusammensetzung, umfassend ein Streptogramin-Derivat der Gruppe A nach Anspruch 1, in reinem Zustand oder in Form einer Assoziation mit mindestens einem Streptogramin-Derivat der Gruppe B und/oder in Form einer Assoziation mit einem oder mehreren kompatiblen und pharmazeutisch akzeptablen Verdünnungsmitteln oder Zusatzstoffen.

9. Pharmazeutische Zusammensetzung nach Anspruch 8, **dadurch gekennzeichnet, daß** das Streptogramin-Derivat der Gruppe B unter den natürlichen Bestandteilen ausgewählt wird: Pristinamycin IA, Pristinamycin IB, Pristinamycin IC, Pristinamycin ID, Pristinamyein IE, Pristinamycin IF, Pristinamycin IG, Virginiamycin S1, S3 oder S4, Vernamycin B oder C, Etamycin oder unter den Derivaten der Hemisynthese der allgemeinen Formel (A): 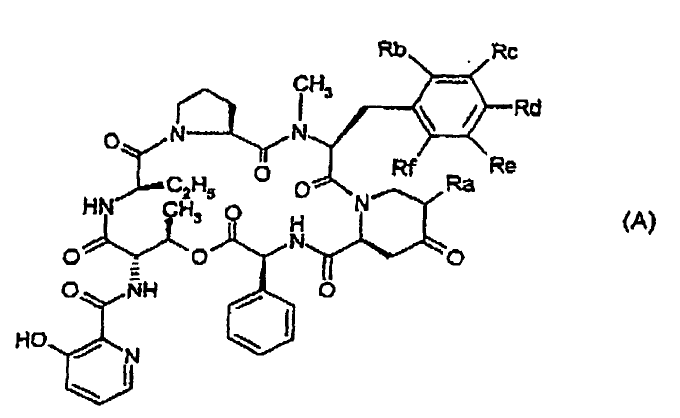 in der
1) Rb, Rc, Re und Rf Wasserstoffatome sind, Rd ein Wasserstoffatom oder ein Rest Dimethylamino ist, und Ra einen Rest der Struktur -CH₂R'a darstellt, worin R'a 3-Pyrrolidinylthio, 3-(oder 4-)Piperidylthio, das substituiert sein kann durch Alkyl, Alkylthio, substituiert durch 1 oder 2 Hydroxysulfonyl, Alkylamino, Dialkylamino (das gegebenenfalls selbst substituiert ist durch Mercapto oder Dialkylamino), oder substituiert durch 1 oder 2 Ringe Piperazin, gegebenenfalls substituiert, Morpholino, Thiomorpholino, Piperidino, 1-Pyrrolidinyl, 2-, 3- oder 4-Piperidyl oder 2- oder 3-Pyrrolidinyl (die substituiert sein können durch Alkyl) bedeutet, oder Ra ist ein Rest der Struktur =CHR'a, worin R'a 3-Pyrrolidinylamino, 3-(oder 4-)Piperidylamino, 3-Pyrrolidinyloxy, 3-(oder 4-)Piperidyloxy, 3-Pyrrolidinylthio, 3-(oder 4-)Piperidylthio ist, die gegebenenfalls substituiert sein können durch Alkyl, oder R'a auch Alkylamino, Alkyloxy, oder Alkylthio, substituiert durch 1 oder 2 Hydroxysulfonyl, Alkylamino, Dialkylamino (das gegebenenfalls selbst substituiert ist durch Dialkylamino), oder durch Trialkylammonio, 4- oder 5-Imidazolyl oder durch 1 oder 2 Ringe Piperazin, gegebenenfalls substituiert, Morpholino, Thiomorpholino, Piperidino, 1-Pyrrolidinyl, 2-, 3- oder 4-Piperidyl oder 2- oder 3-Pyrrolidinyl (die substituiert sein können durch Alkyl) bedeutet, oder
Ra ein Rest 3-(oder 4-)Chinuclidinyl-thiomethyl ist, oder
2) Ra ein Wasserstoffatom ist und
a) entweder Rb, Re und Rf Wasserstoffatome sind, Rd einen Rest -NHCH₃ oder -N(CH₃)₂ darstellt und Rc ein Chloratom oder Bromatom ist oder einen Rest Alkenyl mit 3 bis 5 Kohlenstoffatomen darstellt [wenn Rd -N(CH₃)₂ ist],
b) oder Rb, Rd, Re und Rf ein Wasserstoffatom darstellen und Rc ein Halogen oder ein Rest Aminomonoalkyl, Aminodialkyl, Alkyloxy, Trifluormethyloxy, Thioalkyl, Alkyl mit 1 bis 3 Kohlenstoffatomen oder Trihalogenmethyl ist,
c) oder Rb, Rc, Re und Rf ein Wasserstoffatom darstellen und Rd ein Halogen oder ein Rest Ethylamino, Diethylamino oder Methylethylamino, Alkyloxy oder Trifluormethyloxy, Thioalkyl, Alkyl mit 1 bis 6 Kohlenstoffatomen, Aryl oder Trihalogenmethyl ist,
d) oder Rb, Re und Rf ein Wasserstoffatom darstellen und Rc ein Halogen oder ein Rest Aminomonoalkyl oder Aminodialkyl, Alkyloxy oder Trifluormethyloxy, Thioalkyl, Alkyl mit 1 bis 3 Kohlenstoffatomen ist, und Rd ein Halogen oder ein Rest Amino, Aminomonoalkyl oder Aminodialkyl, Alkyloxy oder Trifluormethyloxy, Thioalkyl, Alkyl mit 1 bis 6 Kohlenstoffatomen oder Trihalogenmethyl ist,
e) oder Rc, Re und Rf ein Wasserstoffatom darstellen und Rb und Rd einen Rest Methyl bedeuten;
oder auch unter den Derivaten der Hemisynthese der allgemeinen Formel (B): 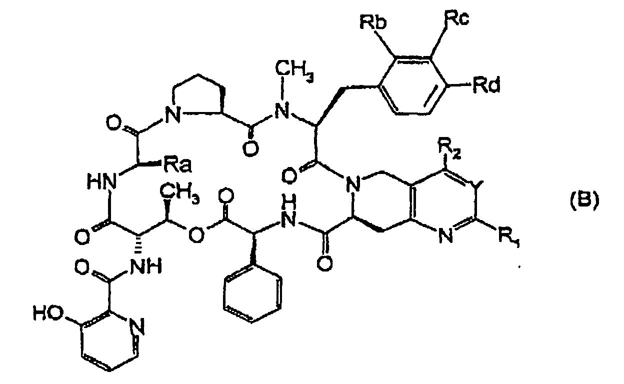 in der
Y ein Stickstoffatom oder ein Rest =CR₃- ist,
R₁ ein Wasserstoffatom, ein Rest Alkyl (1 bis 8 Kohlenstoffatome), Alkenyl (2 bis 8 Kohlenstoffatome), Cycloalkyl (3 bis 8 Kohlenstoffatome), Heterocyclyl, gesättigt oder ungesättigt (3 bis 8 Kettenglieder), Phenyl, Phenyl substituiert [durch ein oder mehrere Halogenatome oder Reste Hydroxy, Alkyl, Alkyloxy, Alkylthio, Alkylsulfinyl, Alkylsulfonyl, Amino, Alkylamino oder Dialkylamino] oder ein Rest NR'R" ist, worin R' und R", gleich oder verschieden, Wasserstoffatome oder Reste Alkyl (1 bis 3 Kohlenstoffatome) sein können, oder auch zusammen mit dem Stickstoffatom, an das sie gebunden sind, einen Heterocyclus mit 3 bis 8 Kettengliedern bilden können, der gegebenenfalls ein anderes Heteroatom enthält, ausgewählt unter Sauerstoff, Schwefel oder Stickstoff, gegebenenfalls substituiert [durch einen Rest Alkyl, Alkenyl (2 bis 8 Kohlenstoffe), Cycloalkyl (3 bis 6 Kohlenstoffe), Heterocyclyl, gesättigt oder ungesättigt (4 bis 6 Kettenglieder), Benzyl, Phenyl oder Phenyl, substituiert wie oben bei der Definition von R₁ angegeben],
oder wenn Y ein Rest =CR₃- ist, R₁ auch Halogenmethyl, Hydroxymethyl, Alkyloxymethyl, Alkylthiomethyl, dessen Teil Alkyl gegebenenfalls substituiert ist durch NR'R", Alkylsulfinylmethyl, Alkylsulfonylmethyl, Acyloxymethyl, Benzoyloxymethyl, Cyclopropylaminomethyl oder -(CH₂)ₙNR'R" (n ist eine ganze Zahl von 1 bis 4 und R' und R" sind wie oben definiert) sein kann, oder auch, wenn R₃ ein Wasserstoffatom ist, R₁ auch Formyl, Carboxy, Alkyloxycarbonyl oder -CONR'R" sein kann, worin R' und R" wie oben definiert sind,
oder wenn Y ein Stickstoffatom ist, R₁ auch ein Rest -XR° sein kann, worin X ein Sauerstoffatom oder Schwefelatom, ein Rest Sulfinyl oder Sulfonyl oder ein Rest NH ist und R° ein Rest Alkyl (1 bis 8 Kohlenstoffe), Cycloalkyl (3 bis 6 Kohlenstoffe), Heterocyclyl, gesättigt oder ungesättigt (3 bis 8 Kettenglieder), Heterocyclylmethyl (3 bis 8 Kettenglieder), dessen Teil Heterocyclyl mit dem Rest Methyl durch ein Kohlenstoffatom verbunden ist, Phenyl, Phenyl substituiert [durch ein oder mehrere Halogenatome oder Reste Alkyl, Alkyloxy, Alkylthio, Alkylsulfinyl, Alkylsulfonyl, Amino, Alkylamino oder Dialkylamino] oder ein Rest -(CH₂)ₙNR'R" ist, worin R' und R" wie oben definiert sind und n eine ganze Zahl von 2 bis 4 darstellt, oder, wenn X NH darstellt, R° auch ein Wasserstoffatom darstellen kann,
R₂ ein Wasserstoffatom oder ein Rest Alkyl (1 bis 3 Kohlenstoffatome) ist,
R₃ ein Wasserstoffatom oder ein Rest Alkyl, Carboxy, Alkyloxycarbonyl oder Carbamoyl der Struktur -CO-NR'R" ist, worin R' und R" wie oben definiert sind,
Ra ein Rest Methyl oder Ethyl ist, und
Rb, Rc und Rd die nachstehenden Definitionen besitzen:
1) Rb und Rc sind Wasserstoffatome und Rd ist ein Wasserstoffatom oder ein Rest Methylamino oder Dimethylamino,
2) Rb ist ein Wasserstoffatom, Rc ist ein Wasserstoffatom, Chloratom oder Bromatom oder stellt einen Rest Alkenyl (3 bis 5 Kohlenstoffe) dar, und Rd ist ein Rest -NMe-R'", worin R'" einen Rest Alkyl, Hydroxyalkyl (2 bis 4 Kohlenstoffe) oder Alkenyl (2 bis 8 Kohlenstoffe), gegebenenfalls substituiert durch Phenyl, Cycloalkyl(3 bis 6 Kohlenstoffe)-methyl, Benzyl, Benzyl substituiert [durch ein oder mehrere Halogenatome oder Reste Hydroxy, Alkyl, Alkyloxy, Alkylthio, Alkylsulfinyl, Alkylsulfonyl, Amino, Alkylamino oder Dialkylamino], Heterocyclylmethyl oder Heterocyclylethyl bedeutet, deren Teil Heterocyclyl gesättigt oder ungesättigt ist und 5 bis 6 Kettenglieder und 1 oder 2 Heteroatome enthält, ausgewählt unter Schwefel, Sauerstoff oder Stickstoff, gegebenenfalls substituiert [einen Rest Alkyl, Alkenyl (2 bis 8 Kohlenstoffe), Cycloalkyl (3 bis 6 Kohlenstoffe), Heterocyclyl, gesättigt oder ungesättigt (4 bis 6 Kettenglieder), Phenyl, Phenyl substituiert, wie vorstehend bei der Definition von R₁ angegeben, oder Benzyl], oder R'" auch einen Rest Cyanomethyl oder -CH₂CORe darstellt, worin entweder Re gleich -OR'e ist und R'e Wasserstoff, Alkyl (1 bis 6 Kohlenstoffe), Alkenyl (2 bis 6 Kohlenstoffe), Benzyl oder Heterocyclylmethyl bedeutet, dessen Teil Heterocyclyl 5 bis 6 Kettenglieder und 1 oder 2 Heteroatome enthält, ausgewählt unter Schwefel, Sauerstoff oder Stickstoff, oder Re ein Rest Alkylamino, Alkylmethylamino, Heterocyclylamino oder Heterocyclylmethylamino darstellt, deren Teil Heterocyclyl gesättigt ist und 5 bis 6 Kettenglieder und 1 oder 2 Heteroatome enthält, ausgewählt unter Schwefel, Sauerstoff oder Stickstoff, gegebenenfalls substituiert durch einen Rest Alkyl, Benzyl oder Alkyloxycarbonyl,
3) Rb ist ein Wasserstoffatom, Rd ist ein Rest -NHCH₃ oder -N(CH₃)₂ und Rc ist ein Chloratom oder Bromatom oder stellt einen Rest Alkenyl (3 bis 5 Kohlenstoffe) dar [wenn Rd gleich -N(CH₃)₂ ist] ,
4) Rb und Rd sind Wasserstoffatome und Rc ist ein Halogenatom oder ein Rest Alkylamino oder Dialkylamino, Alkyloxy, Trifluormethoxy, Thioalkyl, Alkyl (1 bis 6 Kohlenstoffe) oder Trihalogenmethyl,
5) Rb und Rc sind Wasserstoffatome und Rd ist ein Halogenatom oder ein Rest Ethylamino, Diethylamino oder Methylethylamino, Alkyloxy oder Trifluormethoxy, Alkylthio, Alkylsulfinyl, Alkylsulfonyl, Alkyl (1 bis 6 Kohlenstoffe), Phenyl oder Trihalogenmethyl,
6) Rb ist ein Wasserstoffatom und Rc ist ein Halogenatom oder ein Rest Alkylamino oder Dialkylamino, Alkyloxy oder Trifluormethoxy, Thioalkyl, Alkyl (1 bis 3 Kohlenstoffe), und Rd ist ein Halogenatom oder ein Rest Amino, Alkylamino oder Dialkylamino, Alkyloxy oder Trifluormethoxy, Thioalkyl, Alkyl (1 bis 6 Kohlenstoffe) oder Trihalogenmethyl,
7) Rc ist ein Wasserstoffatom und Rb und Rd stellen einen Rest Methyl dar,
sowie ihre Salze,
oder auch unter den Derivaten der Hemisynthese der allgemeinen Formel (C): 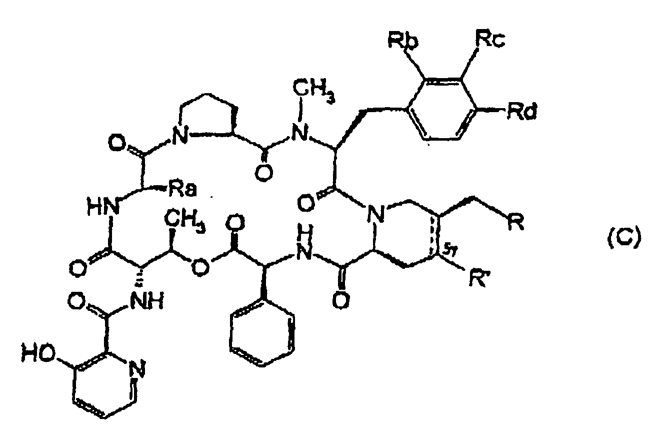 in der
R einen Rest -NR₁R₂ oder -SR₃ darstellt [worin R₁ und R₂, gleich oder verschieden, Wasserstoff, Alkyl (1 bis 8 Kohlenstoffe), gegebenenfalls substituiert durch OH, Alkenyl (3 bis 8 Kohlenstoffe), Cycloalkyl (3 bis 8 Kohlenstoffe) Alkyloxy (1 bis 8 Kohlenstoffe), Dialkylamino, Phenylalkyl, gegebenenfalls substituiert [durch ein oder mehrere Halogenatome oder Alkyl, Hydroxyalkyl, Alkyloxy oder Dialkylamino], Heterocyclylalkyl, gesättigt oder ungesättigt (3 bis 8 Kettenglieder), enthaltend 1 oder mehrere Heteroatome, ausgewählt unter N, S oder O, darstellen, oder R₁ und R₂ zusammen mit dem Stickstoffatom einen mono- oder polycyclischen, gesättigten, partiell gesättigten oder ungesättigten Heterocyclus (3 bis 12 Kettenglieder) bilden, der gegebenenfalls ein anderes Heteroatom enthält, ausgewählt unter N, S oder O, und gegebenenfalls substituiert ist [durch ein oder mehrere OH, Alkyl, Phenyl, gegebenenfalls substituiert durch ein Halogenatom, Phenylalkyl, Phenylalkenyl (Alkenyl enthält 2 bis 4 Kohlenstoffe), Hydroxyalkyl, Acyl, Alkyloxycarbonyl oder Heterocyclyl oder Heterocyclylcarbonyl, dessen Teil Heterocyclyl gesättigt oder ungesättigt ist (4 bis 6 Kettenglieder) und 1 oder mehrere Heteroatome enthält, ausgewählt unter N, S oder O], R₃ Alkyl (1 bis 8 Kohlenstoffe) oder Cycloalkyl (3 bis 8 Kohlenstoffe) ist, substituiert durch -NR₁R₂, worin R₁ und R₂, gleich oder verschieden, Wasserstoff oder Alkyl darstellen oder zusammen mit dem Stickstoffatom, an das sie gebunden sind, ein wie oben definiertes Heterocyclyl bilden, oder auch R₃ Heterocyclyl oder Heterocyclylmethyl, gesättigt oder ungesättigt (3 bis 7 Kettenglieder) darstellt, gegebenenfalls enthaltend ein anderes Heteroatom, ausgewählt unter Sauerstoff, Schwefel oder Stickstoff und gegebenenfalls substituiert durch einen Rest Alkyl; 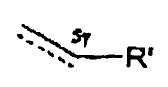 stellt den Rest eines ungesättigten Ringes dar, der in 5γ nicht substituiert ist: 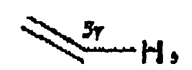 oder den Rest eines gesättigten Ringes, der in 5γ durch einen Rest Fluor substituiert ist: 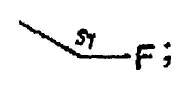 Ra ein Rest Me oder Et ist und Rb, Rc und Rd wie nachstehend definiert sind:
1) Rb und Rc sind Wasserstoff und Rd ist Wasserstoff oder ein Rest MeNH oder NMe₂,
2) Rb ist Wasserstoff, Rc ist Wasserstoff, Chlor oder Brom oder Alkenyl (3 bis 5 Kohlenstoffe) und Rd ist -NMe-R'", worin R'" Alkyl, Hydroxyalkyl (2 bis 4 Kohlenstoffe) oder Alkenyl (2 bis 8 Kohlenstoffe), Phenylalkenyl, Cycloalkyl(3 bis 6 Kohlenstoffe)-methyl, Benzyl, Benzyl substituiert, Heterocyclylmethyl, Heterocyclylethyl, darstellt, oder R'" auch -CH₂CN, -CH₂COOH oder -CORe oder -CH₂CORe bedeutet, worin Re entweder -OR'e ist oder Re Alkylamino, Alkylmethylamino, Heterocyclylamino oder Heterocyclylmethylamino bedeutet,
3) Rb ist Wasserstoff, Rd ist ein Rest -NHCH₃ oder -N(CH₃)₂ und Rc ist Chlor oder Brom, oder Alkenyl (3 bis 5 Kohlenstoffe) [wenn Rd gleich -N(CH₃)₂ ist],
4) Rb und Rd sind Wasserstoff und Rc ist Halogen oder Alkylamino oder Dialkylamino, Alkyloxy, Trifluormethoxy, Thioalkyl, Alkyl (1 bis 6 Kohlenstoffe) oder Trihalogenmethyl,
5) Rb und Rc sind Wasserstoff und Rd ist Halogen oder Ethylamino, Diethylamino oder Methylethylamino, Alkyloxy oder Trifluormethoxy, Alkylthio, Alkylsulfinyl, Alkylsulfonyl, Alkyl (1 bis 6 Kohlenstoffe), Phenyl oder Trihalogenmethyl,
6) Rb ist Wasserstoff und Rc ist Halogen oder Alkylamino oder Dialkylamino, Alkyloxy oder Trifluormethoxy, Thioalkyl, Alkyl (1 bis 3 Kohlenstoffe), und Rd ist Halogen oder ein Rest Amino, Alkylamino oder Dialkylamino, Alkyloxy oder Trifluormethoxy, Thioalkyl, Alkyl (1 bis 6 Kohlenstoffe) oder Trihalogenmethyl,
7) Rc ist Wasserstoff und Rb und Rd sind CH₃,
sowie ihre Salze, wenn sie existieren.
